# EUROPÄISCHE PATENTSCHRIFT

(11) **EP 1 751 101 B1**
(45) Veröffentlichungstag und Bekanntmachung des Hinweises auf die Patenterteilung: **14.11.2012**
(21) Anmeldenummer: 05742882.3
(22) Anmeldetag: 17.05.2005
(51) Int. Cl.: C07D 207/34

(54) **Substituierte 5-Aminomethyl-1H-pyrrol-2-carbonsäureamide**
Substituted 5-aminomethyl-1H-pyrrole-2-carboxylic acid amides
Amides substitués d'acide 5-aminométhyl-1H-pyrrole-2-carboxylique

(30) Priorität: 17.05.2004 DE 102004024772
(43) Veröffentlichungstag der Anmeldung: 14.02.2007
(73) Patentinhaber: Grünenthal GmbH, 52078 Aachen (DE)
(72) Erfinder: MERLA, Beatrix, 52078 Aachen (DE); SUNDERMANN, Corinna, 52074 Aachen (DE); JAGUSCH, Utz-Peter, 52066 Aachen (DE); ENGLBERGER, Werner, 52223 Stolberg (DE); HENNIES, Hagen-Heinrich, 52152 Simmerath (DE); KÖGEL, Babette-Yvonne, 52379 Langerwehe-Hamich (DE)
(74) Vertreter: Brosch, Oliver
(86) Internationale Anmeldenummer: PCT/EP2005/005403
(87) Internationale Veröffentlichungsnummer: WO 2005/113497

(56) Entgegenhaltungen:
- WO-A-99/12933
- WO-A-03/035649
- M. K. SCOTT ET AL.: "Piperazinylalkyl Heterocycles as Potential Antipsychotic Agents" JOURNAL OF MEDICINAL CHEMISTRY., Bd. 38, , Seiten 4198-4210, XP002349085 AMERICAN CHEMICAL SOCIETY. WASHINGTON; US

## Beschreibung

Die vorliegende Erfindung betrifft substituierte 5-Aminomethyl-1H-pyrrol-2-carbonsäureamide, Verfahren zu deren Herstellung, Arzneimittel enthaltend diese Verbindungen sowie die Verwendung dieser Verbindungen zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zieionentierte Behandlung chronischer und nichtchronischer Schmeraustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide, wie beispielsweise das Morphin, sind bei der Therapie starker bis sehr starker Schmerzen wirksam, weisen jedoch unerwünschte Begleiterscheinungen auf, wie beispielsweise Atemdepression, Erbrechen, Sedierung oder Obstipation. Nach weiteren schmerzhemmenden Mitteln wird weitweit geforscht.

WO 99/12933 offenbart Pyrrolopyrrolon-Derivate und deren Verwendung als Inhibitor neutrophiler Elastase. Aus M. K. Scott et al, J. Med.Chem. 1995, 38, 4198-4210 sind Piperazinylalkyl-Heterozyklen und deren Verwendung als Antipsychotika bekannt. WO 03/035649 beschreibt Imidazopyridine als Modulatoren des 5-HT₄ Rezeptors.

Aufgabe der vorliegenden Erfindung war es daher, neue Wirkstoffe zur Verfügung zu stellen, die sich Insbesondere als pharmakologische Wirkstoffe zur Anwendung in Arznelmitteln, vorzugsweise in Arzneimitteln zur Behandlung von Schmerzen eignen.

Diese Aufgabe wurde durch die Bereitstellung der erfindungsgemäßen substituierten 5-Aminomethyt-1H-pyrrol-2-carbonsäureamide der nachstehenden allgemeinen Formel I gelöst.

Es wurde überraschenderweise gefunden, daß die erfindungsgemäßen substituierten 5-Aminomethyl-1H-pyrrol-2-carbonsäureamide der nachstehenden allgemeinen Formel I eine hohe Affinität für Opioid-Rezeptoren, Insbesondere für µ-Opioid-Rezeptoren, aufweisen und sich daher zur Regulation dieser Rezeptoren eignen.

Ferner eignen sich die erfindungsgemäßen substituierten 5-Aminomethyl-1H-pyrrol-2-carbonsäureamide der nachstehenden allgemeinen Formel I zur Regulation, vorzugsweise zur Inhibierung, des Noradrenalin(NA)-Uptakes sowie zur Regulation, vorzugsweise Inhibierung, des 5-Hydroxytryptamin-(5-HT)-Uptakes.

Die erfindungsgemäßen substituierten 5-Aminomethyl-1H-pyrrol-2-carbonsäureamide der nachstehenden allgemeinen Formel I können daher insbesondere als pharmakologische Wirkstoffe in Arzneimitteln zur Prophylaxe und/oder Behandlung von mit den vorstehend genannten Rezeptoren bzw. Prozessen in Verbindung stehenden Störungen oder Erkrankungen eingesetzt werden.

Ein Gegenstand der vorliegenden Erfindung sind daher substituierte 5-Aminomethyl-1H-pyrrol-2-carbonsäureamide der allgemeinen Formel I, 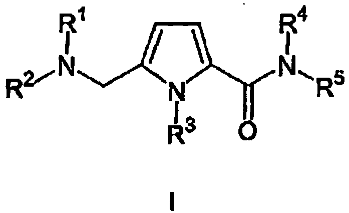 worin
die Reste R¹ und R² zusammen mit dem sie verbindenden Stickstoffatom als Ringglied einen gesättigten oder ungesättigten 4-, 5-, 6-, 7-, 8- oder 9-gliedrigen cycloaliphatischen Rest bilden;
der Rest R³ für einen linearen oder verzweigten, unsubstituierten C₁₋₅-Alkyl-Rest, für einen gesättigten oder ungesättigten, unsubstituierten oder wenigstens einfach substituierten 4-, 5-, 6-, 7-, 8- oder 9-gliedrigen cycloaliphatischen Rest, der über eine lineare oder verzweigte C₁₋₃-AlkylenGruppe gebunden sein kann oder für einen unsubstituierten oder wenigstens einfach substituierten 5- oder 6-gliedrigen Aryl- oder Heteroaryl-Rest, der über eine lineare oder verzweigte C₁₋₃-Alkylen-Gruppe gebunden sein kann, steht,
der Rest R⁴ für einen Wasserstoff-Rest, für einen linearen oder verzweigten, unsubstituierten C₁₋₅-Alkyl-Rest, für einen ungesättigten oder gesättigten, unsubstituierten oder wenigstens einfach substituierten 3-, 4-, 5-, 6-, 7-, 8-oder 9-gliedrigen cycloaliphatischen Rest, oder für einen unsubstituierten oder wenigstens einfach substituierten, über eine lineare oder verzweigte C₁₋₃-Alkylen-Gruppe gebundenen Phenyl-Rest steht,
der Rest R⁵ für einen linearen oder verzweigten, unsubstituierten C₁₋₅-Alkyl-Rest, für einen ungesättigten oder gesättigten, unsubstituierten oder wenigstens einfach substituierten 3-, 4-, 5-, 6-, 7-, 8- oder 9-gliedrigen cycloaliphatischen Rest, oder für einen unsubstituierten oder wenigstens einfach substituierten, über eine lineare oder verzweigte C₁₋₃-Alkylen-Gruppe gebundenen Phenyl-Rest steht,
oder die Reste R⁴ und R⁵ zusammen mit dem sie verbindenden Stickstoffatom als Ringglied einen gesättigten oder ungesättigten, ggf. wenigstens ein weiteres Heteroatom ausgewählt aus der Gruppe bestehend aus Stickstoff, Sauerstoff und Schwefel als Ringglied aufweisenden monozyklischen 4-, 5-, 6-, 7-, 8- oder 9-gliedrigen cycloaliphatischen Rest bilden,
wobei
die entsprechenden Substituenten der vorstehend genannten Aryl-Reste jeweils unabhängig voneinander ausgewählt werden können aus der Gruppe bestehend aus Halogen, Hydroxy, -CN, -CF₃, -CHF₂, -CH₂F, -NO₂, -NH₂, -NH-C₁₋₆-Alkyl, -N(C₁₋₅-Alkyl)₂, -C(-O)-NH₂, -C(=O)-NH-C₁₋₅-Alkyl, -C(=O)-N(C₁₋₅-Alkyl)₂, C₁₋₅-Alkyl, C₁₋₅-Alkoxy und ggf. wenigstens einfach substituiertem Phenyl, wobei, sofern der Phenylsubstituent selbst einfach oder mehrfach, substituiert ist, dessen Substituenten ausgewählt werden können aus der Gruppe bestehend aus Hydroxy, F, Cl, Br, I, -NH₂, -NH-C₁₋₅-Alkyl, -N(C₁₋₅-Alkyl)₂, -C(=O)-NH₂, -C(=O)-NH-C₁-₅-Alkyl, -C(=O)-N(C₁₋₅-Alkyl)₂, -NO₂, -CN, - CF₃, -CHF₂, -CH₂F, C₁₋₅-Alkyl und C₁₋₅-Alkoxy,
die entsprechenden Substituenten der vorstehend genannten Heteroaryl-Reste jeweils unabhängig voneinander ausgewählt werden können aus der Gruppe bestehend aus Halogen, Hydroxy, -CN, -CF₃, -CHF₂, -CH₂F, -NO₂, - NH₂, -NH-C₁₋₅-Alkyl, -N(C₁₋₅-Alkyl)₂, -C(=O)-NH₂, -C(=O)NH-C₁₋₅-Alkyl, -C(=O)-N(C₁₋₅-Alkyl)₂, C₁₋₅-Alkyl, C₁₋₅-Alkoxy und ggf. wenigstens einfach substituiertem Phenyl, wobei, sofern der Phenylsubstituent selbst einfach oder mehrfach, substituiert ist, dessen Substituenten ausgewählt werden können aus der Gruppe bestehend aus Hydroxy, F, Cl, Br, I, -NH₂, -NH-C₁₋₅-Alkyl, - N(C₁₋₅-Alkyl)₂, -C(=O)-NH₂, -C(=O)-NH-C₁₋₅-Alkyl, -C(=O)-N(C₁₋₅-Alkyl)₂, -NO₂, -CN, -CF₃, -CHF₂, -CH₂F, C₁₋₅-Alkyl und C₁₋₅-Alkoxy, und
die vorstehend genannten cycloaliphatischen Reste einfach oder mehrfach, gleich oder verschieden mit einem Substituenten ausgewählt aus der Gruppe bestehend aus C₁₋₅-Alkyl, -C(=O)-O-C₁₋₅-Alkyl, -O-C₁₋₅-Alkyl, Cl, F, Br, I, - C(=O)-NH₂, -C(=O)-NH-C₁₋₅-Alkyl, -C(=O)-N(C₁₋₅-Alkyl)₂, -C(=O)-C₁₋₅-Alkyl, - NH₂, -NH-C₁₋₅-Alkyl, -N(C₁₋₅-Alkyl)₂, Furanyl, Thiophenyl, Pyridinyl, Phenyl und Benzyl substituiert sein können, wobei die zyklischen Substituenten selbst jeweils unsubstituiert oder wenigstens einfach substituiert sein können mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂, - C(=O)-NH₂, -C(-O)-NH-C₁₋₅-Alkyl, -C(=O)-N(C₁₋₅-Alkyl)₂, -NO₂, -CN, -CF₃, - CHF₂, -CH₂F, C₁₋₅-Alkyl und C₁₋₅-Alkoxy;
jeweils gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren, Diastereomeren und/oder Rotameren, in einem beliebigen Mischungsverhältnis, oder jeweils in Form entsprechender Salze, oder jeweils in Form entsprechender Solvate.

Bevorzugt sind erfindungsgemäße substituierte 5-Aminomethyl-1H-pyrrol-2-carbonsäureamide der allgemeinen Formel I, in denen die Reste R¹ und R² zusammen mit dem sie verbindenden Stickstoffatom als Ringglied einen, Aziridinyl-, Azetidinyl-, Pyrrolidinyl-, Piperidinyl-, Azepanyl-, Azokanyl- Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, -O-C₁₋₅-Alkyl, Cl, F, Br, I, -C(=O)-NH₂, -C(=O)-NH-C₁₋₅-Alkyl, -C(=O)-N(C₁₋₅-Alkyl)₂, -C(=O)-C₁-₅-Alkyl, -NH₂, -NH-C₁₋₅-Alkyl, N(C₁₋₅-Alkyl)₂, Furanyl, Thiophenyl, Pyridinyl, Phenyl und Benzyl substituiert sein kann, wobei die zyklischen Substituenten selbst unsubstituiert oder wenigstens einfach substituiert sein können,
und jeweils die Reste R³ bis R⁵ die vorstehend genannte Bedeutung haben, jeweils gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren, Diastereomeren und/oder Rotameren, in einem beliebigen Mischungsverhältnis, oder jeweils in Form entsprechender Salze, oder jeweils In Form entsprechender Solvate.

Weiterhin bevorzugt sind erfindungsgemäße substituierte 5-Aminomethyl-1 H-pyrrol-2-carbonsäureamide der allgemeinen Formel I, in denen R³ für einen Alkyl-Rest ausgewählt aus der Gruppe bestehend aus Methyl, Ethyl, *n*-Propyl, *iso*-Propyl, *n*-Butyl, *iso*-Butyl, *sec*-Butyl und *tert*-Butyl steht, und jeweils die Reste R¹, R², R⁴ und R⁵ die vorstehend genannte Bedeutung haben, Jeweils gegebenenfalls in Form ihrer reinen Stereoisomeren, insbesondere Enantiomeren oder Diastereomeren, ihrer Racemate oder in Form von Mischungen der Stereolsomeren, insbesondere der Enantiomeren, Diastereomeren und/oder Rotameren, in einem beliebigen Mischungsverhältnis, oder jeweils in Form entsprechender Salze, oder jeweils in Form entsprechender Solvate.

Des weiteren sind erfindungsgemäße substituierte 5-Aminomethyl-1H-pyrrol-2-carbonsäureamide der allgemeinen Formel I bevorzugt, in denen R⁴ für einen Wasserstoff-Rest, für einen Alkyl-Rest ausgewählt aus der Gruppe bestehend aus Methyl, Ethyl, *n*-Propyl, *iso*-Propyl, *n-*Butyl, *iso*-Butyl, *sec*-Butyl und *tert*-Butyl, für einen Cyclopropyl-, Cyclobutyl-, Cyclopentyl-, Cyclohexyl-, Cycloheptyl oder Cylcooctyl-Rest oder für einen unsubstituierten oder wenigstens einfach substituierten Benzyl-Rest steht und jeweils die Reste R¹ bis R³ und R⁶ sowie R⁴ und R⁵ zusammen die vorstehend genannte Bedeutung haben, jeweils gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren, Diastereomeren und/oder Rotameren, in einem beliebigen Mischungsverhältnis, oder jeweils in Form entsprechender Salze, oder jeweils in Form entsprechender Solvate.

Ferner sind erfindungsgemäße substituierte 5-Aminomethyl-1H-pyrrol-2-carbonsäureamide der allgemeinen Formel I bevorzugt, in denen R⁵ für einen Alkyl-Rest ausgewählt aus der Gruppe bestehend aus Methyl, Ethyl, *n*-Propyl, *iso*-Propyl, *n*-Butyl, *iso*-Butyl, *sec*-Butyl und *tert*-Butyl, für einen Cyclopropyl-, Cyclobutyl-, Cyclopentyl-, Cyclohexyl, Cycloheptyl oder Cylcooctyl-Rest oder für einen unsubstituierten oder wenigstens einfach substituierten Benzyl-Rest steht, und jeweils die Reste R¹ bis R⁴ sowie R⁴ und R⁵ zusammen die vorstehend genannte Bedeutung haben, jeweils gegebenenfalls in Form ihrer reinen Stereoisomeren, Insbesondere Enantiomeren oder Diastereomeren, ihrer Racemate oder in Form von Mischungen der Stereoisomeren, insbesondere der Enantiomeren, Diastereomeren und/oder Rotameren, in einem beliebigen Mischungsverhältnis, oder jeweils in Form entsprechender Salze, oder jeweils in Form entsprechender Solvate,

Ebenfalls bevorzugt sind erfindungsgemäße substituierte 5-Aminomethyl-1H-pyrrol-2-carbonsäureamide der allgemeinen Formel I, in denen R⁴ und R⁵ zusammen mit dem sie verbindenden Stickstoffatom als Ringglied einen Imidazolidinyl-, Aziridinyl-, Azetidinyl-, Pyrroildinyl-, Piperidinyl-, Azepanyl-, Azokanyl-, Piperazinyl-, Morpholinyl- oder Thiomorpholinyl-Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, -O-C₁₋₅-Alkyl, Cl, F, Br, I, -C(=O)-NH₂, -C(=O)-NH-C₁₋₅-Alkyl, -C(=O)-N(C₁₋₅-Alkyl)₂, -C(=O)-C₁₋₅-Alkyl, -NH₂, -NH-C₁₋₅-Alkyl, -N(C₁₋₅-Alkyl)₂, Furanyl, Thiophenyl, Pyridinyl, Phenyl und Benzyl substituiert sein kann, wobei die zyklischen Substituenten selbst unsubstituiert oder wenigstens einfach substituiert sein können, und jeweils die Reste R¹ bis R³ sowie R⁴ und R⁵ unabhängig voneinander, die vorstehend genannte Bedeutung haben, jeweils gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren, Diastereomeren und/oder Rotameren, in einem beliebigen Mischungsverhältnis, oder jeweils in Form entsprechender Salze, oder jeweils in Form entsprechender Solvate.

Als geeignete Alkyl-, Reste, seien beispielsweise Methyl, Ethyl, n-Propyl, iso-Propyl, n-Butyl, iso-Butyl, sec-Butyl, tert-Butyl, n-Pentyl, iso-Pentyl, neo-Pentyl, -C(H)(C₂H₅)₂, genannt.

Der von R¹ und R² zusammen mit dem sie verbindenden Stickstoffatom als Ringglied gebildete, monozyklische, cycloaliphatische Rest ist gesättigt oder ungesättigt, nicht aber aromatisch, Er kann einfach oder mehrfach, beispielsweise 1-, 2-, 3-, 4- oder 5-fach, gleich oder verschieden substituiert sein, wobei die Substituenten unabhängig voneinander vorzugsweise ausgewählt werden können aus der Gruppe bestehend aus C₁₋₅-Alkyl, -C(=O)-O-C₁₋₅-Alkyl, -O-C₁₋₅-Alkyl, Cl, F, Br, I, -C(=O)-NH_{2.} -C(=O)-NH-C₁₋₅-Alkyl, -C(=O)-N(C₁₋₅-Alkyl)₂, -C(=O)-C₁₋₅-Alkyl, -NH₂, -NH-C₁₋₅-Alkyl, -N(C₁₋₅-Alkyl)₂, Furanyl, Thiophenyl, Phenyl-, Pyridinyl und Benzyl, besonders bevorzugt aus der Gruppe bestehend aus Methyl, Ethyl, *n*-Propyl, *iso*-Propyl, -C(=O)-O-CH₃, - C(=O)-O-C₂H₅, -C(=O)-O-(*n*-C₃H₇), -C(=O)-O-(*iso*-C₃H₇) und ggf. über eine -(CH₂)-Gruppe gebundenem Phenyl. Der jeweilige Furanyl-, Thiophenyl-, Pyridinyl-, Phenyl-oder Benzyl-Substituent kann selbst im Ringsystem einfach oder mehrfach, ggf. 1-, 2-, 3-, 4- oder 5-fach substituiert sein, wobei dessen Substituenten unabhängig voneinander bevorzugt ausgewählt werden können aus der Gruppe bestehend aus Hydroxy, F, Cl, Br, I, -NH₂, -NH-C₁₋₅-Alkyl, -N(C₁₋₅-Alkyl)₂, -C(=O)-NH₂, -C(=O)-NH-C₁₋₆-Alkyl, -C(=O)-N(C₁₋₅-Alkyl)₂, -NO₂, -CN, -CF₃, -CHF₂, -CH₂F, C₁₋₅-Alkyl und C₁₋₅-Alkoxy.

Sofern die Reste R⁴ und R⁵ zusammen mit dem sie verbindenden Stickstoffatom als Ringglied einen ggf. wenigstens ein weiteres Heteroatom als Ringglied aufweisenden cycloaliphatischen Rest bilden, kann dieser monozyklische Rest gesättigt oder ungesättigt, nicht aber aromatisch sein, Er kann einfach oder mehrfach, beispielsweise 1-, 2-, 3-, 4- oder 5-fach, gleich oder verschieden substituiert sein, wobei die Substituenten unabhängig voneinander vorzugsweise ausgewählt werden können aus der Gruppe bestehend aus C₁₋₅-Alkyl, -C(=O)-O-C₁₋₅-Alkyl, -O-C₁₋₅-Alkyl, Cl, F, Br, I, -C(=O)-NH₂, -C(=O)-NH-C₁₋₅-Alkyl, -C(=O)-N(C₁₋₅-Alkyl)₂, -C(=O)-C₁₋₅-Alkyl, -NH₂, -NH-C₁₋₅-Alkyl, -N(C₁₋₅-Alkyl)₂, Furanyl, Thiophenyl, Pyridinyl, Phenyl-und Benzyl, besonders bevorzugt aus der Gruppe bestehend aus Methyl, Ethyl, *n-*Propyl, *iso*-Propyl, -C(=O)-O-CH₃, -C(=O)-O-C₂H₅, -C(=O)-O-(*n*-C₃H₇), -C(=O)-O-(*iso*-C₃H₇) und ggf. über eine -(CH₂)-Gruppe gebundenem Phenyl. Der jeweilige Furanyl-, Thiophenyl-, Pyridinyl-, Phenyl- oder Benzyl-Substituent kann selbst einfach oder mehrfach, ggf. 1-, 2-, 3-, 4- oder 5-fach, substituiert sein, wobei dessen Substituenten unabhängig voneinander bevorzugt ausgewählt werden können aus der Gruppe bestehend aus Hydroxy, F, Cl, Br**,** I, -NH₂, -NH-C₁₋₅-Alkyl, -N(C₁₋₅-Alkyl)₂, -C(=O)-NH₂, -C(=O)-NH-C₁₋₅-Alkyl, -C(=O)-N(C₁₋₅-Alkyl)₂, -NO₂, -CN, -CF₃, -CHF₂, - CH₂F, C₁₋₅-Alkyl und C₁₋₅-Alkoxy.

Sofern die Reste R⁴ und R⁵ zusammen mit dem sie verbindenden Stickstoffatom als Ringglied einen cycloaliphatischen Rest bilden, der eines oder mehrere, beispielsweise 1, 2 oder 3, Heteroatome als Ringglieder aufweist, können die Heteroatome, sofern nicht anders angegeben, unabhängig voneinander bevorzugt ausgewählt werden aus der Gruppe bestehend aus Sauerstoff, Stickstoff und Schwefel.

Sofern die R¹ und R² und/oder die Reste R⁴ und R⁵ zusammen mit dem sie verbindenden Stickstoffatom als Ringglied einen ggf. wenigstens ein weiteres Heteroatom als Ringglied aufweisenden cycloaliphatischen Rest bilden, der einfach oder mehrfach, beispielsweise 1-, 2-, 3-, 4- oder 5-fach substituiert sein kann, kann dieser bevorzugt ausgewählt werden aus der Gruppe bestehend aus Imidazolidinyl, Aziridinyl, Azetidinyl, Pyrrolidinyl, Piperidinyl, Azepanyl, Azokanyl, Piperazinyl, Morpholinyl und Thiomorpholinyl.

Sofern einer oder mehrere der vorstehend genannten Reste R³ bis R⁵ für einen gesättigten oder ungesättigten, cycloaliphatischen Rest stehen oder einen solchen Rest aufweisen, der einfach oder mehrfach, ggf. 1-, 2-, 3-, 4- oder 5-fach, gleich oder verschieden substituiert Ist, so können die entsprechenden Substituenten unabhängig voneinander vorzugsweise ausgewählt werden aus der Gruppe bestehend aus C₁₋₅-Alkyl, -C(=O)-O-C₁₋₅-Alkyl, -O-C₁₋₅-Alkyl, Cl, F, Br, I, -C(=O)-NH₂, -C(=O)-NH-C₁₋₅-Alkyl, -C(=O)-N(C₁₋₅-Alkyl)₂, -C(=O)-C₁₋₅-Alkyl, -NH₂, -NH-C₁₋₅-Alkyl, -N(C₁₋₅-Alkyl)₂ Furanyl, Thiophenol, Pyridinyl, Phenyl- und Benzyl, besonders bevorzugt aus der Gruppe bestehend aus Methyl, Ethyl, *n*-Propyl, *iso*-Propyl, -C(=O)-O-CH₃, -C(=O)-O-C₂H₅, -C(=O)-O-(*n*-C₃H₇), -C(=O)-O-(*iso*-C₃H₇) und ggf. über eine -(CH₂)-Gruppe gebundenem Phenyl. Sofern der Furanyl-, Thiophenyl-, Pyridinyl-, Phenyl- oder Benzyl-Substituent selbst einfach oder mehrfach, ggf. 1-, 2-, 3-, 4- oder 5-fach, substituiert ist, können dessen Substituenten unabhängig vonelnander bevorzugt ausgewählt werden aus der Gruppe bestehend aus Hydroxy, F, Cl, Br, I, -NH₂, -NH-C₁₋₅-Alkyl, -N(C₁₋₅-Alkyl)₂, -C(=O)-NH₂, -C(=O)-NH-C₁₋₅-Alkyl, -C(=O)-N(C₁₋₅-Alkyl)₂, - NO₂, -CN, -CF₃, -CHF₂, -CH₂F, C₁₋₅-Alkyl und C₁₋₅-Alkoxy.

Als geeignete cycloaliphatische Reste, die einfach oder mehrfach, ggf. 1-, 2-, 3-, 4-oder 5-fach substituiert sein können, seien beispielsweise Cyclopropyl, Cyclobutyl, Cyclopentyl, Cyclohexyl, Cycloheptyl, Cyclooctyl, Cyclononyl, Cyclopentenyl Cyclohexenyl, Cycloheptonyl und Cyclooctenyl genannt.

Sofern einer oder mehrere der vorstehend genannten Reste R³ bis R⁵ einen einfach oder mehrfach, beispielsweise 1-, 2-, 3-, 4- oder 5-fach, substituierten Aryl-Rest aufweisen, so können die entsprechenden Substituenten jeweils unabhängig voneinander vorzugsweise ausgewählt werden aus der Gruppe bestehend aus Halogen, Hydroxy, -CN, -CF₃, -CHF₂, -CH₂F, -NO₂, -NH₂, -NH-C₁₋₅-Alkyl. -N(C₁₋₅-Alkyl)₂, -C(-O)-NH₂, -C(=O)-NH-C₁₋₅-Alkyl, -C(=O)-N(C₁₋₅-Alkyl)₂, C₁₋₅-Alkyl, C₁₋₅-Alkoxy und ggf. wenigstens einfach substituiertem Phenyl, besonders bevorzugt aus der Gruppe bestehend aus F, Cl, Br, Hydroxy, -OCH₃ und -CH₃. Sofern der Phenylsubstituent selbst einfach oder mehrfach, beispielsweise 1-, 2-, 3-, 4- oder 5-fach, substituiert ist, können dessen Substituenten bevorzugt ausgewählt werden aus der Gruppe bestehend aus Hydroxy, F, Cl, Br, I, -NH₂, -NH-C₁₋₅-Alkyl, -N(C₁₋₅-Alkyl)₂, -C(=O)-NH₂,-C(=O)-NH-C₁₋₅-Alkyl, -C(=O)-N(C₁₋₅-Alkyl)₂, -NO₂, -CN, -CF₃, -CHF₂, - CH₂F, C₁₋₅-Alkyl und C₁₋₅-Alkoxy.

Als geeignete Aryl-Reste, die einfach oder mehrfach substituiert sein können, seien beispielsweise Phenyl genannt.

Sofern einer oder mehrere der vorstehend genannten Reste R³ bis R⁵ einen einfach oder mehrfach, beispielsweise 1-, 2-, 3-, 4- oder 5-fach, substituierten Heteroaryl-Rest aufweisen, so können die entsprechenden Substituenten jeweils unabhängig voneinander vorzugsweise ausgewählt werden aus der Gruppe bestehend aus Halogen, Hydroxy, -CN, -CF₃, -CHF₂, -CH₂F, -NO₂, -NH₂, -NH-C₁₋₅-Alkyl, -N(C₁₋₅-Alkyl)₂, -C(=O)-NH₂, -C(=O)-NH-C₁₋₅-Alkyl, -C(=O)-N(C₁₋₅-Alkyl)₂, C₁₋₅-Alkyl, C₁₋₅-Alkoxy und ggf. wenigstens einfach substituiertem Phenyl, besonders bevorzugt aus der Gruppe bestehend aus F, Cl, Br, Hydroxy, -OCH₃ und -CH₃. Sofern der Phenylsubstituent selbst einfach oder mehrfach, beispielsweise 1-, 2-, 3-, 4- oder 5-fach, substituiert ist, können dessen Substituenten bevorzugt ausgewählt werden aus der Gruppe bestehend aus Hydroxy, F, Cl, Br, I, -NH₂, -NH-C₁₋₅-Alkyl, -N(C₁₋₅-Alkyl)₂, -C(=O)-NH₂, -C(=O)-NH-C₁₋₅-Alkyl, -C(=O)-N(C₁₋₅-Alkyl)₂, -NO₂, -CN, -CF₃, -CHF₂, - CH₂F, C₁₋₅-Alkyl und C₁₋₅-Alkoxy. Vorzugsweise ist der Heteroaryl-Rest monozyklisch und enthalt ein, zwei oder drei Heteroatome ausgewählt aus der Gruppe bestehend aus Sauerstoff, Schwefel und Stickstoff.

Als geeignete Heteroaryl-Reste seien beispielsweise Pyrrolyl, Furyl (Furanyl), Thienyl (Thiophenyl), Pyrazolyl, Imidazolyl, Thiazolyl, Thiadiazolyl, Triazolyl, Oxazolyl, Isoxazolyl, Pyridinyl, Pyridazinyl, Pyrimidinyl, Pyrazinyl, Pyranyl, genannt.

Die vorstehend genannten C₁₋₅-Alkyl- und C₁₋₅-Alkoxy-Reste können jeweils linear oder verzweigt sein. Die C₁₋₅-Alkyl-Reste umfassen die Reste Methyl, Ethyl, n-Propyl, iso-Propyl, n-Butyl, iso-Butyl, sec-Butyl, tert-Butyl, n-Pentyl, iso-Pentyl und neo-Pentyl, die C₁₋₅-Alkoxy-Reste umfassen die Reste Methoxy, Ethoxy, n-Propoxy, iso-Propoxy, n-Butoxy, iso-Butoxy, sec-Butoxy, tert-Butoxy, n-Pentoxy, iso-Pentoxy und neo-Pentoxy.

Ganz besonders bevorzugt sind erfindungsgemäße substituierte 5-Aminomethyl-1H-pyrrol-2-carbonsäureamide ausgewählt aus der Gruppe bestehend aus
1-Methyl-5-(4-methyl-piperidin-1-ylmethyl)-1H-pyrrol-2-carbonsäure-benzylmethylamid,
(5-Azepan-1-ylmethyl-1-methyl-1H-pyrrol-2-yl)-pyrrolidin-1-yl-methanon,
[1-Methyl-5-(2-methyl-piperidin-1-ylmethyl)-1H-pyrrol-2-yl]-pyrrolidin-1-ylmethanon,
1-Methyl-5-pyrrolidin-1-ylmethyl-1H-pyrrol-2-carbonsäurebenzylamid,
1-Methyl-5-(2-methyl-piperidin-1-ylmethyl)-1H-pyrrol-2-carbonsäurebenzyl-methylamid,
1-(5-Dibenzylcarbamoyl-1-methyl-1H-pyrrol-2-ylmethyl)-piperidin-4-carbonsäureethylester,
5-(4-Benzyl-piperidin-1-ylmethyl)-1-methyl-1H-pyrrol-2-carbonsäurecyclohexylmethylamid,
[5-(4-Benzyl-piperidin-1-ylmethyl)-1-methyl-1H-pyrrol-2-yl]-piperidin-1-ylmethanon,
1-Methyl-5-(2-methyl-piperidin-1-ylmethyl)-1H-pyrrol-2-carbonsäurebenzylisopropylamid und
[1-Methyl-5-(2-methyl-piperidin-1-ylmethyl)-1H-pyrrol-2-yl]-piperidin-1-ylmethanon,
jeweils gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren, Diastereomeren und/oder Rotameren, in einem beliebigen Mischungsverhältnis, oder jeweils in Form entsprechender Salze, oder jeweils in Form entsprechender Solvate.

Ein weiterer Gegenstand der vorliegenden Erfindung ist ein Verfahren zur Herstellung der erfindungsgemäßen substituierten 5-Aminomethyl-1H-pyrrol-2-carbonsäureamide der allgemeinen Formel I, gemäß dem ein substituiertes 1H-pyrrol-2-carbonsäureamid der allgemeinen Formel II 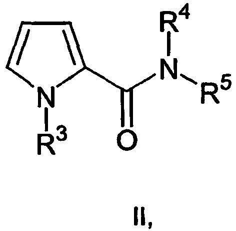 worin die Reste R³, R⁴ und R⁵ die vorstehend genannte Bedeutung haben, nach üblichen, dem Fachmann bekannten Methoden, vorzugsweise in einem geeigneten Reaktionsmedium, wie z.B. CH₂Cl₂, CH₃CN, Dimethylformamid (DMF) oder Mischungen aus wenigstens zwei dieser Komponenten, bei Raumtemperatur (ca. 20-25°C) mit einem Iminiumsalz der allgemeinen Formel III, 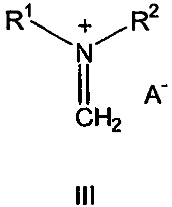 worin R¹ bis R² die vorstehend genannte Bedeutung haben und A⁻ für ein geeignetes Anion, vorzugsweise für Cl⁻, AlCl₄⁻, Br⁻, I⁻ oder CF₃-SO₃⁻ (Triflat-Anion) steht, zu einem erfindungsgemäßen substituierten 5-Aminomethyl-1H-pyrrol-2-carbonsäureamid der allgemeinen Formel I umgesetzt und dieses ggf. nach üblichen, dem Fachmann bekannten Methoden, vorzugsweise durch Extraktion, gereinigt und ggf. isoliert wird.

Die Verbindungen der allgemeinen Formel II können nach üblichen, dem Fachmann bekannten Methoden, beispielsweise aus den am Markt käuflich erhältlichen Reagenzien der allgemeinen Formel IV 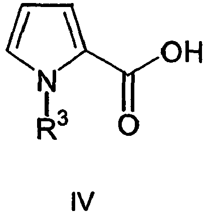 hergestellt werden, wie z.B. in A.J. Carpenter, D.J. Chadwick, Journal of Organic Chemistry, 1985, 50, Seiten 4362-4368 und in T.J. Donohoe, P. M. Guyo, Journal of Organic Chemistry 1996, 61, Seiten 7664-7665 beschrieben. Die entsprechende Literaturbeschreibung wird hiermit als Referenz eingeführt und gilt als Teil der Offenbarung.

Die Iminiumsalze der allgemeinen Formel III können ebenfalls nach üblichen, dem Fachmann bekannten Verfahren, beispielsweise aus den entsprechenden Aminalen der nachstehenden allgemeinen Formel V 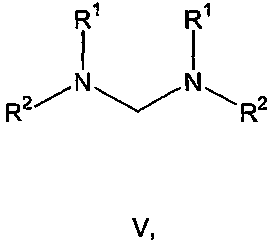 worin die Reste R¹ und R² die vorstehend genannte Bedeutung haben, erhalten werden, wie beispielsweise in H. Heaney, Tetrahedron 1997, 53, Seiten 2941-2958 und H. Heaney, Tetrahedron Lett. 1988, 29, Seiten 2377-2380 beschrieben. Die entsprechenden Literaturbeschreibungen werden hiermit als Referenz eingeführt und gelten als Teil der Offenbarung.

Auch die Herstellung der Aminale der allgemeinen Formel V kann nach literaturbekannten Methoden erfolgen, wie z.B. in H. Heaney, Tetrahedron 1997, 53, Seiten 2941-2958 und H. Heaney, Tetrahedron Lett. 1988, 29, Seiten 2377-2380 beschrieben. Die entsprechenden Literaturbeschreibungen werden hiermit als Referenz eingeführt und gelten als Teil der Offenbarung.

Die erfindungsgemäßen substituierten 5-Aminomethyl-1H-pyrrol-2-carbonsäureamide der allgemeinen Formel I sowie entsprechende Stereoisomere können sowohl in Form ihrer freien Basen, ihrer freien Säuren wie auch jeweils in Form entsprechender Salze, insbesondere physiologisch verträglicher Salze, isoliert werden.

Die freien Basen der jeweiligen erfindungsgemäßen 5-Aminomethyl-1H-pyrrol-2-carbonsäureamide der allgemeinen Formel I sowie entsprechender Stereoisomere können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in die entsprechenden Salze, vorzugsweise physiologisch verträglichen Salze, überführt werden.

Die freien Basen der jeweiligen erfindungsgemäßen 5-Aminomethyl-1H-pyrrol-2-carbonsäureamide der allgemeinen Formel I sowie entsprechender Stereoisomere können ebenfalls bevorzugt durch Versetzen der in einem geeigneten organischen Lösungsmittel, wie z.B. Butan-2-on (Methylethylketon), gelösten Verbindungen der allgemeinen Formel I oder entsprechender Stereoisomere als freie Basen mit Trimethylsilylchlorid (TMSCI) in die entsprechenden Hydrochloride übergeführt werden.

Die freien Basen der jeweiligen 5-Aminomethyl-1H-pyrrol-2-carbonsäureamide der allgemeinen Formel I und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der jeweiligen 5-Aminomethyl-1H-pyrrol-2-carbonsäureamide der allgemeinen Formel I und entsprechender Stereoisomere durch Umsetzung mit einer geeigneten Base in die entsprechenden Salze, insbesondere physiologisch verträglichen Salze überführt werden.

Die erfindungsgemäßen 5-Aminomethyl-1H-pyrrol-2-carbonsäureamide der allgemeinen Formel I und entsprechende Stereoisomere können ggf., ebenso wie jeweils die entsprechenden Säuren, die entsprechenden Basen oder Salze dieser Verbindungen, nach üblichem, dem Fachmann bekannten Methoden auch in Form ihrer Solvate, vorzugsweise ihrer Hydrate, erhalten werden.

Sofern die erfindungsgemäßen substituierten 5-Aminomethyl-1H-pyrrol-2-carbonsäureamide der allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren und/oder Rotameren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an stationärer chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten 5-Aminomethyl-1H-pyrrol-2-carbonsäureamide der allgemeinen Formel I und entsprechende Stereoisomere sowie jeweils die entsprechenden Säuren, Basen, Salze und Solvate sind toxikologisch unbedenklich und eignen sich als pharmazeutische Wirkstoffe in Arzneimitteln.

Ein weiterer Gegenstand der vorliegenden Erfindung sind daher Arzneimittel enthaltend wenigstens ein erfindungsgemäßes substituiertes 5-Aminomethyl-1H-pyrrol-2-carbonsäureamid der allgemeinen Formel I, gegebenenfalls in Form seines Racemates, eines seiner reinen Stereoisomeren, insbesondere Enantiomeren oder Diastereomeren, oder in Form von Mischungen der Stereoisomeren, insbesondere der Enantiomeren, Diastereomeren und/oder Rotameren, in einem beliebigen Mischungsverhältnis, oder jeweils in Form entsprechender Salze, insbesondere der physiologisch verträglichen Salze, besonders bevorzugt der Hydrochloride, oder entsprechender Solvate, insbesondere der Hydrate, sowie ggf. physiologisch verträgliche Hilfsstoffe.

Diese erfindungsgemäßen Arzneimittel eignen sich insbesondere zur Opioid-Rezeptor-Regulation, vorzugsweise zur µ-Opioid-Rezeptor-Regulation, zur Regulation des Noradrenalin(NA)-Uptakes, vorzugsweise zur Inhibierung des Noradrenalin(NA)-Uptakes, sowie zur Regulation des 5-Hydroxytryptamin-(5-HT)-Uptakes, vorzugsweise zur Inhibierung des 5-Hydroxytryptamin-(5-HT)-Uptakes.

Ebenfalls bevorzugt eignen sich die erfindungsgemäßen Arzneimittel zur Prophylaxe und/oder Behandlung von Störungen oder Krankheiten, die zumindest teilweise durch Opioid-Rezeptoren, insbesondere µ-Opioid-Rezeptoren, und/oder Noradrenalin(NA)-Rezeptoren und/oder 5-Hydroxytryptamin-(5-HT)-Rezeptoren vermittelt werden.

Ebenfalls bevorzugt eignen sich die erfindungsgemäßen Arzneimittel zur Bekämpfung von Schmerzen, vorzugsweise ausgewählt aus der Gruppe bestehend aus chronischen Schmerzen, akuten Schmerzen und neuropathischen Schmerzen, zur Prophylaxe und/oder Behandlung von Entzugserscheinungen, Gedächtnisstörungen, neurodegenerativen Erkrankungen, vorzugsweise ausgewählt aus der Gruppe bestehend aus Morbus Parkinson, Morbus Huntington, Morbus Alzheimer und Multipler Sklerose, Epilepsie, cardiovaskulären Störungen, Wasserspeicher-Krankheiten, intestinaler Motilität (Diarrhoe), Harninkontinenz, Anorexie, Tinnitus, Pruritus, Depressionen, sexueller Dysfunktionen, vorzugsweiser erektiler Dysfunktion oder Atemwegserkrankungen, zur Prophylaxe und/oder Behandlung von Störungen der Nahrungsmittelaufnahme, vorzugsweise ausgewählt aus der Gruppe bestehend aus Fettsucht, Bulimie, Anorexie, Kachexie und Type II Diabetes (nicht-insulinabhängiger Diabetes) oder zur Anxiolyse, zur Diurese, zur Unterdrückung des Miktionsreflexes, zur Reduzierung des Suchtpotentials von Opioiden, vorzugsweise Morphin, zur Modulation der Bewegungsaktivität, zur Beeinflussung des cardiovaskulären Systems, vorzugsweise zur Vasodilatation der Arterien, oder zur Regulation des Elektrolyt-Haushaltes.

Besonders bevorzugt eignen sich die erfindungsgemäßen Arzneimittel zur Behandlung von Schmerzen, vorzugsweise ausgewählt aus der Gruppe bestehend aus chronischen Schmerzen, akuten Schmerzen und neuropathischen Schmerzen.

Ein weiterer Gegenstand der vorliegenden Erfindung ist die Verwendung eines oder mehrerer substituierter 5-Aminomethyl-1H-pyrrol-2-carbonsäureamide der allgemeinen Formel I, jeweils gegebenenfalls in Form ihrer reinen Stereoisomeren, inbesondere Enantiomeren oder Diastereomeren, ihrer Racemate oder in Form von Mischungen der Stereoisomeren, insbesondere der Enantiomeren, Diastereomeren und/oder Rotameren, in einem beliebigen Mischungsverhältnis, oder jeweils in Form entspechender Salze, insbesondere der physiologisch verträglichen Salze, besonders bevorzugt der Hydrochloride, oder jeweils in Form entsprechender Solvate, insbesondere der Hydrate, zur Herstellung eines Arzneimittels zur Opiod-Rezeptor-Regulation, vorzugsweise zur Regulation des µ-Opioid-Rezeptors, zur Regulation des Noradrenalin(NA)-Uptakes, vorzugsweise zur Inhibierung des Noradrenalin(NA)-Uptakes oder zur Regulation des Hydroxytryptamin-(5-HT)-Uptakes, vorzugsweise zur Inhibierung des 5-Hydroxytryptamin-(5-HT)-Uptakes.

Ein weiterer Gegenstand der vorliegenden Erfindung ist die Verwendung eines oder mehrerer substituierter 5-Aminomethyl-1H-pyrrol-2-carbonsäureamide der allgemeinen Formel I, jeweils gegebenenfalls in Form ihrer reinen Stereoisomeren, inbesondere Enantiomeren oder Diastereomeren, ihrer Racemate oder in Form von Mischungen der Stereoisomeren, insbesondere der Enantiomeren, Diastereomeren und/oder Rotameren, in einem beliebigen Mischungsverhältnis, oder jeweils in Form enstsprechender Salze, insbesondere der physiologisch verträglichen Salze, besonders bevorzugt der Hydrochloride, oder jeweils in Form entsprechender Solvate, insbesondere der Hydrate, zur Herstellung eines Arzneimittels zur Bekämpfung von Schmerzen, vorzugsweise ausgewählt aus der Gruppe bestehend aus chronischen Schmerzen, akuten Schmerzen und neuropathischen Schmerzen, zur Prophylaxe und/oder Behandlung von Entzugserscheinungen, Gedächtnisstörungen, neurodegenerativen Erkrankungen, vorzugsweise ausgewählt aus der Gruppe bestehend aus Morbus Parkinson, Morbus Huntington, Morbus Alzheimer und/oder multipler Sklerose, Epilepsie, cardiovaskulärer Störungen, Wasserspeicher-Krankheiten, intestinaler Motilität (Diarrhoe), Haminkontinenz, Anorexie, Tinnitus, Pruritus, Depressionen, sexueller Dysfunktionen, vorzugsweiser erektiler Dysfunktion oder Atemwegserkrankungen, zur Prophylaxe und/oder Behandlung von Störungen der Nahrungsmittelaufnahme, vorzugsweise ausgewählt aus der Gruppe bestehend aus Fettsucht, Bulimie, Anorexie, Kachexie und Type II Diabetes (nicht-insulinabhängiger Diabetes), oder zur Anxiolyse, zur Diurese, zur Unterdrückung des Miktionsreflexes, zur Reduzierung des Suchtpotentials von Opioiden, vorzugsweise Morphin, zur Modulation der Bewegungsaktivität, zur Beeinflussung des cardiovaskulären Systems, vorzugsweise zur Vasodilatation der Arterien, oder zur Regulation des Elektrolyt-Haushaltes

Die erfindungsgemäßen Arzneimittel können als flüssige, halbfeste oder feste Arzneiformen, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben einem oder mehreren substituierten 5-Aminomethyl-1H-pyrrol-2-carbonsäureamiden der allgemeinen Formel I, jeweils gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren, Diastereomeren und/oder Rotatmeren, in einem beliebigen Mischungsverhältnis, oder jeweils in Form eines entsprechenden Salzes, insbesondere eines physiologisch verträglichen Salzes, oder in Form eines entsprechenden Solvates, insbesondere des Hydrates, enthalten die erfindungsgemäßen Arzneimittel üblicherweise weitere physiologisch verträgliche pharmazeutische Hilfsstoffe, die bevorzugt ausgewählt sind aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll.

Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Substituierte 5-Aminomethyl-1H-pyrrol-2-carbonsäureamide der allgemeinen Formel I, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren, Diastereomeren und/oder Rotameren, in einem beliebigen Mischungsverhältnis, oder jeweils in Form eines entsprechenden Salzes, insbesondere eines physiologisch verträglichen Salzes, oder in Form eines entsprechenden Solvates, insbesondere des Hydrates, in einem Depot in gelöster Form oder in einem Pflaster, gegebenenfalls unter Zusatz von die Hautpenetration fördernden Mitteln, sind geeignete perkutane Applikationszubereitungen.

Oral oder perkutan anwendbare Zubereitungsformen können die jeweiligen substituierten 5-Aminomethyl-1H-pyrrol-2-carbonsäureamide der allgemeinen Formel I, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren, Diastereomeren und/oder Rotameren, in einem beliebigen Mischungsverhältnis, oder jeweils in Form eines entsprechenden Salzes, insbesondere eines physiologisch verträglichen Salzes, oder in Form eines entsprechenden Solvates, insbesondere des Hydrates, auch verzögert freisetzen.

Die Herstellung der erfindungsgemäßen Arzneimittel erfolgt mit Hilfe von üblichen, dem Fachmann bekannten Mitteln, Vorrichtungen, Methoden und Verfahren, wie sie beispielsweise in A.R. Gennaro (Hrsg.), Remington's Pharmaceutical Sciences, 17. Edition, Mack Publishing Company, Easton, Pa. (1985), insbesondere in Teil 8, Kapitel 76 bis 93, beschrieben sind. Die entsprechende Literaturbeschreibung wird hiermit als Referenz eingeführt und gilt als Teil der Offenbarung.

Die an den Patienten zu verabreichende Menge des jeweiligen substituierten 5-Aminomethyl-1H-pyrrol-2-carbonsäureamids der allgemeinen Formel I, gegebenenfalls in Form des Racemates, der reinen Stereoisomeren, insbesondere Enantiomeren oder Diastereomeren, oder in Form von Mischungen der Stereoisomeren, insbesondere der Enantiomeren, Diastereomeren und/oder Rotameren, in einem beliebigen Mischungsverhältnis, oder jeweils in Form eines entsprechenden Salzes, insbesondere eines physiologisch verträglichen Salzes, oder jeweils in Form eines entsprechenden Solvates, insbesondere des Hydrates, kann variieren und ist beispielsweise abhängig vom Gewicht oder Alter des Patienten sowie von der Applikationsart, der Indikation und dem Schweregrad der Erkrankung. Üblicherweise werden 0,005 bis 500 mg/kg, vorzugsweise 0,05 bis 50 mg/kg Körpergewicht des Patienten wenigstens eines substituierten 5-Aminomethyl-1H-pyrrol-2-carbonsäureamids der allgemeinen Formel I, gegebenenfalls in Form seines Racemates, seines reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form eines entsprechenden Salzes, insbesondere eines physiologisch verträglichen Salzes, oder in Form eines entsprechenden Solvates, insbesondere des Hydrates, appliziert.

### Pharmakologische Methoden:

### a) Methode zur Bestimmung der Affinität zum humanen µ-Opioidrezeptor

Die Rezeptoraffinität zum humanen µ-Opioidrezeptor wird in einem homogenen Ansatz in Mikrotiterplatten bestimmt. Hierzu werden Verdünnungsreihen des jeweils zu prüfenden substituierten 5-Aminomethyl-1H-pyrrol-2-carbonsäureamids der allgemeinen Formel I mit einer Rezeptormembranpräparation (15-40 µg Protein pro 250 µl Inkubationsansatz) von CHO-K1-Zellen, welche den humanen µ-Opioidrezeptor (µ-Opiatrezeptor) exprimieren (RB-HOM-RezeptormembranPräparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wird 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wird zusätzlich 25 µmol/l Naloxon zugegeben. Nach Beendigung der neunzigminütigen Inkubationszeit werden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wird die prozentuale Verdrängung des radioaktiven Liganden aus seiner Bindung zum humanen µ-Opiatrezeptor bei einer Konzentration der zu prüfenden Verbindungen von 1 µmo/l bestimmt und als Prozent Hemmung der spezifischen Bindung angegeben. Ausgehend von der prozentualen Verdrängung durch unterschiedliche Konzentrationen der zu prüfenden Verbindungen der allgemeinen Formel I wurden IC₅₀ Hemmkonzentrationen berechnet, die eine 50-prozentige Verdrängung des radioaktiven Liganden bewirken. Durch Umrechnung mittels der Cheng-Prusoff-Beziehung lassen sich Kᵢ-Werte für die Prüfsubstanzen erhalten.

### b) Methode zur Bestimmung der Noradrenalin- und der 5HT-Uptake-Inhibierung:

Für in vitro Studien werden Synaptosomen aus Rattenhimarealen frisch isoliert, wie in der Veröffentlichung "The isolation of nerve endings from brain" von E.G. Gray und V.P. Whittaker, J. Anatomy 96, Seiten 79-88, 1962, beschrieben. Die entsprechende Literaturbeschreibung wird hiermit als Referenz eingeführt und gilt als Teil der Offenbarung.

Das Gewebe (Hypothalamus für die Bestimmung der Noradrenalin-Uptake-Inhibierung und Mark und Pons für die Bestimmung der 5HT-Uptake-Inhibierung) wird in eisgekühlter 0,32 M Sucrose (100 mg Gewebe / 1 mL) in einem Glas-Homogenisierer mit Teflonstößel homogenisiert, indem fünf volle Auf- und Abschläge bei 840 Umdrehungen /Minute benutzt werden.

Das Homogenat wird bei 4°C für 10 Minuten bei 1000 g zentrifugiert. Nach anschließender Zentrifugierung bei 17000 g für 55 Minuten erhält man die Synaptosomen (P₂-Fraktion), die in 0,32 M Glucose (0,5 mU 100 mg des ursprünglichen Gewichts) noch einmal suspendiert werden.
Der jeweilige Uptake wird in einer 96-well Mikrotiterplatte gemessen. Das Volumen beträgt 250 µl und die Inkubation erfolgt bei Raumtemperatur (ca. 20-25 °C) unter O₂ Atmosphäre.

Die Inkubationszeit beträgt 7,5 Minuten für [³H]-NA und 5 Minuten für [³H]-5-HT. Anschließend werden die 96 Proben durch eine Unifilter GF/B^{®} Mikrotiterplatte (Packard) filtriert und mit 200 mL inkubierten Puffer mit Hilfe eines "Brabdel Cell-Harvester MPXRI-96T" gewaschen. Die Unifilter GF/B Platte wird bei 55°C 1 Stunde getrocknet. Im Anschluß wird die Platte mit einem Back seal^{®} (Packard) verschlossen und mit 35 µl Szintilationsflüssigkeit pro Well (Ultima Gold^{®}, Packard) versetzt. Nach dem Verschließen mit einem top seal^{®} (Packard) wird, nach der Einstellung des Gleichgewichts (etwa 5 Stunden), die Radioaktivität in einem "Trilux 1450 Microbeta" (Wallac, Freiburg, Deutschland) bestimmt.

Die Menge des bei der vorstehenden Bestimmung eingesetzten Proteins entspricht den aus der Literatur bekannten Werten, wie z.B. in "Protein measurement with the folin phenol reagent", Lowry et al., J. Biol. Chem., 193, 265-275, 1951 beschrieben.

Eine detaillierte Methodenbeschreibung kann ferner auch der Literatur, beispielsweise aus M.Ch. Frink, H.-H. Hennies, W. Engelberger, M. Haurand und B. Wilffert (1996) Arzneim.-Forsch./Drug Res. 46 (III), 11, 1029-1036, entnommen werden.

Die entsprechenden Literaturbeschreibungen werden hiermit jeweils als Referenz eingeführt und gelten als Teil der vorliegenden Offenbarung.

Folgende Kenndaten wurden für den NA- bzw. 5-HT-Transporter ermittelt:
- NA-Uptake :: Km = 0,32 ± 0,11 µM
- 5HT-Uptake :: Km = 0,084 ± 0,011 µM

### c) Untersuchung auf analgetische Wirksamkeit im Writhing-Test

Die Untersuchung der erfindungsgemäßen Verbindungen der allgemeinen Formel I auf analgetische Wirksamkeit wird im Phenylchinon-induzierten Writhing an der Maus, modifiziert nach I.C. Hendershot und J. Forsaith (1959) J. Pharmacol. Exp. Ther. 125, 237-240 durchgeführt. Die entsprechende Literaturbeschreibung wird hiermit als Referenz eingeführt und gilt als Teil der Offenbarung.

Dazu werden männliche NMRI-Mäuse mit einem Gewicht von 25 bis 30 g verwendet. Gruppen von 10 Tieren pro Verbindungsdosis erhalten 10 Minuten nach intravenöser Gabe der zu untersuchenden Verbindungen 0,3 ml/Maus einer 0,02%igen wäßrigen Lösung von Phenylchinon (Phenylbenzochinon, Fa. Sigma, Deisenhofen, Deutschland; Herstellung der Lösung unter Zusatz von 5 Gew.-% Ethanol und Aufbewahrung im Wasserbad bei 45°C) intraperitoneal appliziert. Die Tiere werden einzeln in Beobachtungskäfige gesetzt. Mit Hilfe eines Drucktastenzählers wird die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung erhalten. Alle Verbindungen werden in der Standarddosierung von 10 mg/kg getestet.

Im folgenden wird die Erfindung anhand von Beispielen erläutert. Diese Erläuterungen sind lediglich beispielhaft und schränken den allgemeinen Erfindungsgedanken nicht ein.

### Beispiele:

Die Messung der NMR-Spektren erfolgte auf einem Gerät vom Typ Bruker DPX 300 für die 300 MHz-Spektren und auf einem Gerät vom Typ Bruker DRX 600 für die 600 MHz-Spektren.

Die jeweiligen Chemikalien und Lösungsmittel wurden kommerziell von den üblichen Herstellern erworben.

### A)

Allgemeine Synthesevorschrift zur Herstellung von Verbindungen der allgemeinen Formel II:

Die jeweilige 1H-Methyl-pyrrol-2-carbonsäure (50 mmol) der allgemeinen Formel IV (worin R³ jeweils für eine Methyl-Gruppe steht) wurde in 200 ml Toluol suspendiert. Anschließend wurden unter Kühlung im Eisbad langsam 100 mmol Natriumhydrid (NaH) zugegeben und für circa 10 Minuten bei Raumtemperatur gerührt. Nach Zugabe von 200 mmol Oxalylchlorid wurde das Reaktionsgemisch für 15 Minuten unter Rückfluß erhitzt und anschließend das überschüssige Oxalylchlorid sowie das Reaktionsmedium am Rotationsverdampfer entfernt Der so erhaltene Rückstand wurde in 200 ml Diethylether aufgenommen, langsam mit 100 mmol des entsprechenden Amins der allgemeinen Formel NHR⁴R⁵ versetzt und das so erhaltene Reaktionsgemisch zwei Stunden unter Rückfluß erhitzt. Nach dem Abkühlen wurde das Reaktionsgemisch dreimal mit jeweils 50 ml Wasser gewaschen. Die organische Phase wurde abgetrennt, über Magnesiumsulfat getrocknet und am Rotationsverdampfer eingeengt. Das so erhaltene Rohprodukt wurde ohne weitere Aufreinigung in den nachfolgenden Syntheseschritten eingesetzt.

Die nach der vorstehenden allgemeine Synthesevorschrift hergestellten Verbindungen der allgemeinen Formel II sind in der nachfolgenden Tabelle 1 wiedergegeben:

| [1] | R⁴ | R⁵ | R³ | Ausbeute [%] |
|---|---|---|---|---|
| **II-1** | CH₂-Phenyl | CH₂-Phenyl | CH₃ | 90 |
| **II-2** | ]-(CH₂)₅-[ | | CH₃ | 82 |
| **II-3** | ]-(CH₂)₄-[ | | CH₃ | 74 |
| **II-4** | CH₂-Phenyl | CH₃ | CH₃ | 93 |
| **II-5** | CH₂-Phenyl | CH(CH₃)₂ | CH₃ | 93 |
| **II-6** | Cyclohexyl | CH₃ | CH₃ | 89 |

### [1]: Verbindung der allgemeinen Formel II

Die Struktur der Verbindungen **II-1** bis **II-6** wurde jeweils mit Hilfe der ¹H-NMR-Spektroskopie bestimmt. Im folgenden sind die chemischen Verschiebungen ausgewählter Verbindungen wiedergegeben.

### II-1 1-Methyl-1H-pyrrol-2-carbonsäure-dibenzylamid

δ (DMSO, 300 MHz) = 3,73 (s, 3 H, NC*H*₃); 4,58 - 4,72 (m, 4 H, C*H*₂Ph); 5,95 (dd, 1 H, *J* = 2,6 Hz, *J* = 6,0 Hz, N(CH₃)]-CHC*H*C*H*C-[); 6,23 - 6,29 (m, 1 H, N(CH₃)-]-CHC*H*C*H*C-[); 6,88 - 6,93 (m, 1 H, N(CH₃)-]-CHC*H*C*H*C-[); 7,16 - 7,42 (m, 10 H, Ph).

### II-2 (1-Methyl-1H-pyrrol-2-yl)-piperidin-1-yl-methanon

δ (DMSO, 300 MHz) = 1,45 - 1,68 (m, 6 H, N-[(C*H*₂)₂-C*H*₂-(C*H*₂)₂-[); 3,51 - 3,62 (m, 4 H, N-[(C*H*₂)₂-CH₂-(C*H*₂)₂-[); 3,65 (s, 3 H, NC*H*₃); 5,97 - 6,03 (m, 1 H, N(CH₃)-]-CHC*H*C*H*C-[); 6,21 - 6,25 (m, 1 H, N(CH₃)]-CHC*H*C*H*C-[); 6,79 - 6,84 (m, 1 H, N(CH₃)]-C*H*C*H*CHC-[).

### II-4 1-Methy/-1H-pyrro/-2-carbonsäure-benzyl-methy/-amid

δ (DMSO, 300 MHz) = 2,99 (br. s, 3 H, NC*H*₃Bn); 3,71 (s, 3 H, NC*H*₃); 4,65 - 4,75 (m, 2 H, C*H*₂Ph); 5,96 - 6,04 (m, 1 H, N(CH₃)]-CHC*H*C*H*C-[); 6,28 - 6,41 (m, 1 H, N(CH₃)-]-CHC*H*C*H*C-[); 6,87 - 6,93 (m, 1 H, N(CH₃)-]-CHC*H*C*H*C-[); 7,18 - 7,43 (m, 5 H, Ph).

### II-5 1-Methyl-1H-pyrrol-2-carbonsäure-benzyl-isopropyl-amid

δ (DMSO, 300 MHz) = 1,15 (d, 3 H, *J*= 6,8 Hz, CH(C*H*₃)₂); 3,67 (s, 3 H, NC*H*₃); 4,52 - 4,67 (m, 3 H, C*H*₂Ph, CH(C*H*₃)₂); 5,95 - 6,03 (m, 1 H, N(CH₃)]-CHC*H*C*H*C-[); 6,25 - 6,34 (m, 1 H, N(CH₃)-]-CHC*H*C*H*C-[); 6,80 - 6,86 (m, 1 H, N(CH₃)-]-C*H*C*H*C*H*C-[); 7,15 - 7,36 (m, 5 H, Ph).

### II-6 1-Methyl-1H-pyrrol-2-carbonsäure-cyclohexyl-methyl-amid

δ (DMSO, 300 MHz) = 0,88 - 1,36 (m, 4 H, N]-CH(CH₂)₂(C*H*₂)₂C*H*₂-[); 1,44 - 1,85 (m, 6 H, N]-CH(C*H*₂)₂(CH₂)₂CH₂-[); 2,88 (s, 3 H, N-CH₃); 3,64 (s, 3 H, NC*H*₃); 4,03 - 4,19 (m, 1 H, N]-C*H*(CH₂)₂(CH₂)₂C*H*₂-[); 5,97 - 6,04 (m, 1 H, N(CH₃)]-CHC*H*C*H*C-[); 6,19 - 6,29 (m, 1 H, N(CH₃)]-CHC*H*C*H*C-[); 6,79 - 6,85 (m, 1 H, N(CH₃)]-CHC*H*C*H*C-[).

### B)

Allgemeine Herstellungsvorschrift für die automatisierte Synthese der erfindungsgemäßen Verbindungen der allgemeinen Formel I. Die Synthese wurde auf einer automatisierten Syntheseanlage der Firma Zymark durchgeführt.
- Ansatzmengen:: • 300µmol Aminal der allgemeinen Formel V
• 300µmol Pyrrol der allgemeinen Formel II
• 600µmol Acetylchlorid
- Pipettiervolumina:: • 1ml Aminallösung
• 1ml Pyrrollösung
• 0,6ml Acetylchloridlösung
- Stammlösungen:: • 0,3M Aminallösung in CH₃CN (Lösung I)
• 0,3M Pyrrollösung in CH₃CN (Lösung II)
• 1M Acetylchloridlösung in CH₃CN (Lösung III)

### Synthesedurchführung:

In ein trockenes Gewindeglas mit Septumkappe wurden bei 20°C 300 µmol Aminal-Verbindung der allgemeinen Formel V (Lösung I, 1 ml) vorgelegt und mit 600 µmol Acetylchlorid (Lösung III, 0,6 ml) versetzt. Nach 1 Stunde wurde 300 µmol Pyrrol-Derivat der allgemeinen Formel II (Lösung II, 1 ml) zupipettiert. Die Reaktionslösung wurde 24 Stunden bei 20°C gerührt. Anschließend wurden in der Quench-Station bei Raumtemperatur 2 ml HCl-Lösung (0.1 M) und 2 ml CH₂Cl₂ zugegeben. Die erhaltene Lösung wurde 30 Minuten im Spin-Reaktor durchmischt. Der Rührfisch wurde entfernt und das Gefäß mit 2ml CH₂Cl₂ ausgespült.

### Syntheseaufarbeitung:

Die untere, organische Phase wurde abgenommen und verworfen. Im Vortexer wurden 4 ml CH₂Cl₂ zugegeben und anschließend weitere 10 Minuten im Spinreaktor durchmischt. Die organische Phase wurde nach dem Zentrifugieren wieder abgenommen und verworfen, die wäßrige Phase wurde nochmals mit 4 ml CH₂Cl₂ versetzt und mit 0,7 ml 7,5%iger NaHCO₃-Lösung auf pH 8-9 eingestellt. Die Lösung wurde im Spinreaktor intensiv durchmischt, und nach dem Zentrifugieren wurde die organische Phase abgetrennt und gesammelt. Die wäßrige Phase wurde analog noch ein Mal mit 4 ml CH₂Cl₂ extrahiert. Anschließend wurden die vereinigten, organischen Phasen über eine MgSO₄-Kartusche getrocknet und unter vermindertem Druck eingeengt. Die weitere Reinigung der Verbindungen erfolgte mittels präparativer HPLC.

Die folgenden erfindungsgemäßen Beispielverbindungen der allgemeinen Formel I wurden so hergestellt:

### Beispiel 1:

### 1-Methyl-5-(4-methyl-piperidin-1-ylmethyl)-1H-pyrrole-2-carbonsäure-benzyl-methyl-amid

MS: (ESI) m/z = 339 [M⁺ + 1]; 241.

### Beispiel 2:

### (5-Azepan-1-ylmethyl-1-methyl-1H-pyn-ol-2-yl)-pyrrolidin-1-yl-methanon

MS: (ESI) m/z = 290 [M⁺ + 1]; 219; 191

### Beispiel 3:

### [1-Methyl-5-(2-methyl-piperidin-1-ylmethyl)-1H-pyrrol-2-yl]-pyrrolidin-1-yl-methanon

MS: (ESI) m/z = 290 [M⁺ + 1]; 191.

### Beispiel 4:

### 1-Methyl-5-pyrrolidin-1-ylmethyl-1H-pyrrol-2-carbonsäurebenzylamid

MS: (ESI) m/z = 312 [M⁺ + 1]; 241.

### Beispiel 5:

### 1-Methyl-5-(2-methyl-piperidin-1-ylmethyl)-1H-pyrrol-2-carbonsäurebenzyl-methyl-amid

MS: (ESI) m/z = 340 [M⁺ +1]; 241.

### Beispiel 6:

### 1-(5-Dibenzylcarbamoyl-1-methyl-1H-pyrrol-2-ylmethyl)-piperidin-4-carbonsäureeth ylester

MS: (ESI) m/z = 475 [M⁺ +1]; 317; 198.

### Beispiel 7:

### 5-(4-Benzyl-piperidin-1-ylmethyl)-1-methyl-1H-pyrrol-2-carbonsäurecyclohexyl-methyl-amid

MS: (ESI) m/z = 409 [M⁺ +1]; 233.

### Beispiel 8:

### [5-(4-Benzyl-piperidin-1-ylmethyl)-1-methyl-1H-pyrrol-2-yl]-piperidin-1-yl-methanon

MS: (ESI) m/z = 381 [M⁺ +1]; 205.

### Beispiel 9:

### 1-Methyl-5-(2-methyl-piperidin-1-ylmethyl)-1H-pyrrol-2-carbonsäurebenryl-isopropyl-amid

MS: (ESI) m/z = 368 [M⁺ +1]; 269.

### Beispiel 10:

### [1-Methyl-5-(2-methyl-piperidin-1-ylmethyl)-1H-pyrrol-2-yl]-piperidin-1-yl-methanon

MS: (ESI) m/z = 304 [M⁺ + 1]; 205.

### Pharmakologische Untersuchungen:

### a) Affinität zum µ-Rezeptor

Die Affinität der erfindungsgemäßen 5-Aminomethyl-1H-pyrrol-2-carbonsäureamide zum µ-Opioid-Rezeptor wurde wie vorstehend beschrieben bestimmt. Die Werte für einige ausgewählte Verbindungen sind in der nachfolgenden Tabelle 1 wiedergegeben.

**Tabelle 1:**

| Verbindung gemäß Beispiel | R¹ | R² | R⁴ | R⁵ | R³ | ORµ1-human Naloxon Inhibierung [%] |
|---|---|---|---|---|---|---|
| **3** | ]-(CH₂)₅-[-2-CH₃ | | ]-(CH₂)₄-[ | | CH₃ | **83** |
| **10** | ]-(CH₂)₅-[-2-CH₃ | | ]-(CH₂)₅-[ | | CH₃ | **86** |

### b) 5-HT-Uptake-Inhibierung und Noradrenalin(NA)-Uptake-Inhibierung

Die 5-HT-Wiederaufnahmehemmung sowie die Noradrenalin-Wiederaufnahmehemmung der erfindungsgemäßen 5-Aminomethyl-1H-pyrrol-2-carbonsäureamide wurde wie vorstehend beschrieben bestimmt. Die Werte für einige ausgewählte Verbindungen sind in der nachfolgenden Tabelle 2 wiedergegeben.

**Tabelle 2:**

| Verbindung gemäß Beispiel | R¹ | R² | R⁴ | R⁵ | R³ | 5HT-Uptake Hemmung [2] | NA-Uptake Hemmung [3] |
|---|---|---|---|---|---|---|---|
| **1** | ]-(CH₂)₅-[-4-CH₃ | | CH₃ | CH₂-Phenyl | CH₃ | 70 | 81 |
| **2** | ]-(CH₂)₆-[ | | ]-(CH₂)₄-[ | | CH₃ | 60 | |
| **3** | ]-(CH₂)₅-[-2-CH₃ | | ]-(CH₂)₄-[ | | CH₃ | 52 | |
| **4** | ]-(CH₂)₄-[ | | CH₃ | CH₂-Phenyl | CH₃ | | 82 |
| **5** | ]-(CH₂)₅-[-2-CH₃ | | CH₃ | CH₂-Phenyl | CH₃ | | 81 |
| **6** | ]-(CH₂)₅-[-4-CO₂CH₂CH₃ | | CH₂-Phenyl | CH₂-Phenyl | CH₃ | | 74 |
| **7** | ]-(CH₂)₅-[-4-CH₂Phenyl | | CH₃ | Cyclohexyl | CH₃ | | 62 |
| **8** | ]-(CH₂)₅-[-4-CH₂Phenyl | | ]-(CH₂)₅-[ | | CH₃ | | 61 |
| **9** | ]-(CH₂)₅-[-2-CH₃ | | CH₂-Phenyl | CH(CH₃)₂ | CH₃ | | 59 |

[2] 5-HT-Uptake, Ratte, 10 µM,% Hemmung

[3] Na-Uptake, Ratte, 10 µM, % Hemmung

## Patentansprüche

1. Substituierte 5-Aminomethyl-1 H-pyrrol-2-carbonsäureamide der allgemeinen Formel I, 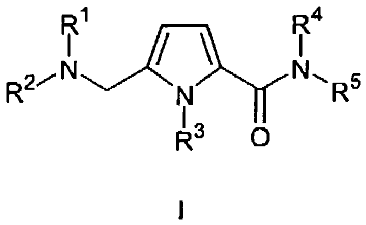 worin
die Reste R¹ und R² zusammen mit dem sie verbindenden Stickstoffatom als Ringglied einen gesättigten oder ungesättigten 4-, 5-, 6-, 7-, 8- oder 9-gliedrigen cycloaliphatischen Rest bilden;
der Rest R³ für einen linearen oder verzweigten, unsubstituierten C₁₋₅-Alkyl-Rest, für einen gesättigten oder ungesättigten, unsubstituierten oder wenigstens einfach substituierten 4-, 5-, 6-, 7-, 8- oder 9-gliedrigen cycloaliphatischen Rest, der über eine lineare oder verzweigte C₁₋₃-AlkylenGruppe gebunden sein kann oder für einen unsubstituierten oder wenigstens einfach substituierten 5- oder 6-gliedrigen Aryl- oder Heteroaryl-Rest, der über eine lineare oder verzweigte C₁₋₃-Alkylen-Gruppe gebunden sein kann, steht,
der Rest R⁴ für einen Wasserstoff-Rest, für einen linearen oder verzweigten, unsubstituierten C₁₋₅-Alkyl-Rest, für einen ungesättigten oder gesättigten, unsubstituierten oder wenigstens einfach substituierten 3-, 4-, 5-, 6-, 7-, 8-oder 9-gliedrigen cycloaliphatischen Rest, oder für einen unsubstituierten oder wenigstens einfach substituierten, über eine lineare oder verzweigte C₁₋₃-Alkylen-Gruppe gebundenen Phenyl-Rest steht,
der Rest R⁵ für einen linearen oder verzweigten, unsubstituierten C₁₋₅-Alkyl-Rest, für einen ungesättigten oder gesättigten, unsubstituierten oder
wenigstens einfach substituierten 3-, 4-, 5-, 6-, 7-, 8- oder 9-gliedrigen cycloaliphatischen Rest, oder für einen unsubstituierten oder wenigstens einfach substituierten, über eine lineare oder verzweigte C₁₋₃-Alkylen-Gruppe gebundenen Phenyl-Rest steht,
oder die Reste R⁴ und R⁵ zusammen mit dem sie verbindenden Stickstoffatom als Ringglied einen gesättigten oder ungesättigten, ggf. wenigstens ein weiteres Heteroatom ausgewählt aus der Gruppe bestehend aus Stickstoff, Sauerstoff und Schwefel als Ringglied aufweisenden monozyklischen 4-, 5-, 6-, 7-, 8- oder 9-gliedrigen cycloaliphatischen Rest bilden,
wobei
die entsprechenden Substituenten der vorstehend genannten Aryl-Reste jeweils unabhängig voneinander ausgewählt werden können aus der Gruppe bestehend aus Halogen, Hydroxy, -CN, -CF₃, -CHF₂, -CH₂F, -NO₂, -NH₂, -NH-C₁₋₆-Alkyl, -N(C₁₋₅-Alkyl)₂, -C(=O)-NH₂, -C(=O)-NH-C₁₋₅-Alkyl, -C(=O)-N(C₁₋₅-Alkyl)₂, C₁₋₅-Alkyl, C₁₋₅-Alkoxy und ggf. wenigstens einfach substituiertem Phenyl, wobei, sofern der Phenylsubstituent selbst einfach oder mehrfach, substituiert ist, dessen Substituenten ausgewählt werden können aus der Gruppe bestehend aus Hydroxy, F, Cl, Br, I, -NH₂, -NH-C₁₋₅-Alkyl, -N(C₁₋₅-Alkyl)₂, -C(=O)-NH₂, -C(=O)-NH-C₁₋₅-Alkyl, -C(=O)-N(_{C1-5}-Alkyl)₂, -NO₂, -CN, - CF₃, -CHF₂, -CH₂F, C₁₋₅-Alkyl und C₁₋₅-Alkoxy,
die entsprechenden Substituenten der vorstehend genannten Heteroaryl-Reste jeweils unabhängig voneinander ausgewählt werden können aus der Gruppe bestehend aus Halogen, Hydroxy, -CN, -CF₃, -CHF₂, -CH₂F, -NO₂, - NH₂. -NH-C₁₋₅-Alkyl, -N(C₁₋₆-Alkyl)₂, -C(=O)-NH₂. -C(=O)-NH-C₁₋₅-Alkyl, -C(=O)-N(C₁₋₅-Nkyl)₂, C₁₋₅-Alkyl, C₁₋₅-Alkoxy und ggf. wenigstens einfach substituiertem Phenyl, wobei, sofern der Phenylsubstituent selbst einfach oder mehrfach, substituiert ist, dessen Substituenten ausgewählt werden können aus der Gruppe bestehend aus Hydroxy, F, Cl, Br, I, -NH₂, -NH-C₁₋₅-Alkyl, - N(C₁₋₅-Alkyl)₂, -C(=O)-NH₂, -C(=O)-NH-C₁₋₅-Alkyl, -C(=O)-N(C₁₋₅-Alkyl)₂, -NO₂, -CN, -CF₃, -CHF₂, -CH₂F, C₁₋₅-Alkyl und C₁₋₅-Alkoxy, und
die vorstehend genannten cycloaliphatischen Reste einfach oder mehrfach, gleich oder verschieden mit einem Substituenten ausgewählt aus der Gruppe bestehend aus C₁₋₅-Alkyl, -C(=O)-O-C₁₋₅-Alkyl, -O-C₁₋₅-Alkyl, Cl, F, Br, I,-C(=O)-NH₂, -C(=O)-NH-C₁₋₅-Alkyl, -C(=O)-N(C₁₋₅-Alkyl)₂, -C(=O)-C₁₋₅-Alkyl, - NH₂, -NH-C₁₋₅-Alkyl, -N(C₁₋₅-Alkyl)₂, Furanyl, Thiophenyl, Pyridinyl, Phenyl und Benzyl substituiert sein können, wobei die zyklischen Substituenten selbst jeweils unsubstituiert oder wenigstens einfach substituiert sein können mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂, - C(=O)-NH₂, -C(=O)-NH-C₁₋₅-Alkyl, -C(=O)-N(C₁₋₅-Alkyl)₂, -NO₂, -CN, -CF₃, - CHF₂, -CH₂F, C₁₋₅-Alkyl und C₁₋₅-Alkoxy,
jeweils gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren, Diastereomeren und/oder Rotameren, in einem beliebigen Mischungsverhältnis, oder jeweils in Form entsprechender Salze, oder jeweils in Form entsprechender Solvate.

2. Verbindungen gemäß Anspruch 1, **dadurch gekennzeichnet, daß** die Reste R¹ und R² zusammen mit dem sie verbindenden Stickstoffatom als Ringglied einen Aziridinyl-, Azetidinyl-, Pyrrolidinyl-, Piperidinyl-, Azepanyl- oder Azokanyl-Rest bilden, der einfach oder mehrfach, gleich oder verschieden mit einem Substituenten ausgewählt aus der Gruppe bestehend aus C₁₋₅-Alkyl, - C(=O)-O-C₁₋₅-Alkyl, -O-C₁₋₅-Alkyl, Cl, F, Br, I, -C(=O)-NH₂,-C(=O)-NH-C₁₋₅-Alkyl, -C(=O)-N(C₁₋₅-Alkyl)₂, -C(=O)-C₁₋₅-Alkyl, -NH₂, -NH-C₁₋₅-Alkyl, -N(C₁₋₅-Alkyl)₂, Furanyl, Thiophenyl, Pyridinyl, Phenyl und Benzyl substituiert sein kann, wobei die zyklischen Substituenten selbst jeweils unsubstituiert oder wenigstens einfach substituiert sein können.

3. Verbindungen gemäß Anspruch 1 oder 2, **dadurch gekennzeichnet, daß** der Rest R³ für einen Alkyl-Rest ausgewählt aus der Gruppe bestehend aus Methyl, Ethyl, *n*-Propyl, *iso*-Propyl, *n*-Butyl, *iso*-Butyl, *sec*-Butyl und *tert*-Butyl steht.

4. Verbindungen gemäß einem oder mehreren der Ansprüche 1 bis 3, **dadurch gekennzeichnet, daß** der Rest R⁴ für einen Wasserstoff-Rest, für einen Alkyl-Rest ausgewählt aus der Gruppe bestehend aus Methyl, Ethyl, *n*-Propyl, *iso-*Propyl, *n*-Butyl, *iso*-Butyl, *sec*-Butyl und *tert*-Butyl, für einen Cyclopropyl-, Cyclobutyl-, Cyclopentyl-, Cyclohexyl-, Cycloheptyl oder Cylcooctyl-Rest oder für einen unsubstituierten oder wenigstens einfach substituierten Benzyl-Rest steht.

5. Verbindungen gemäß einem oder mehreren der Ansprüche 1 bis 4, **dadurch gekennzeichnet, daß** der Rest R⁵ für einen Alkyl-Rest ausgewählt aus der Gruppe bestehend aus Methyl, Ethyl, *n*-Propyl, *iso*-Propyl, *n*-Butyl, *iso*-Butyl, *sec*-Butyl und *tert*-Butyl, für einen Cyclopropyl-, Cyclobutyl-, Cyclopentyl-, Cyclohexyl-, Cycloheptyl oder Cylcooctyl-Rest oder für einen unsubstituierten oder wenigstens einfach substituierten Benzyl-Rest steht.

6. Verbindungen gemäß einem oder mehreren der Ansprüche 1 bis 5, **dadurch gekennzeichnet, daß** die Reste R⁴ und R⁵ zusammen mit dem sie verbindenden Stickstoffatom als Ringglied einen Imidazolidinyl-, Aziridinyl-, Azetidinyl-, Pyrrolidinyl-, Piperidinyl-, Azepanyl-, Azokanyl-, Piperazinyl-, Morpholinyl- oder Thiomorpholinyl-Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, -O-C₁₋₅-Alkyl, Cl, F, Br, I, - C(=O)-NH₂, -C(=O)-NH-C₁₋₅-Alkyl, -C(=O)-N(C₁₋₅-Alkyl)₂, -C(=O)-C₁₋₅-Alkyl, - NH₂, -NH-C₁₋₅-Alkyl, -N(C₁₋₅-Alkyl)₂, Furanyl, Thiophenyl, Pyridinyl, Phenyl und Benzyl substituiert sein kann, wobei die zyklischen Substituenten selbst jeweils unsubstituiert oder wenigstens einfach substituiert sein können.

7. Verbindungen gemäß einem oder mehreren der Ansprüche 1 bis 6, **dadurch gekennzeichnet, daß**
R¹ und R² zusammen mit dem sie verbindenden Stickstoffatom als Ringglied einen Aziridinyl-, Azetidinyl-, Pyrrolidinyl-, Piperidinyl-, Azepanyl- oder Azokanyl-Rest bilden, der mit einem Substituenten ausgewählt aus der Gruppe bestehend aus Methyl, Ethyl, *n*-Propyl, *iso*-Propyl, -C(=O)-O-CH₃, - C(=O)-O-C₂H₅, -C(=O)-O-(*n*-C₃H₇), -C(=O)-O-(*iso*-C₃H₇) und ggf. über eine - (CH₂)-Gruppe gebundenem Phenyl substituiert sein kann,
R³ für einen Alkyl-Rest ausgewählt aus der Gruppe bestehend aus Methyl, Ethyl, *n*-Propyl, *iso*-Propyl, *n*-Butyl, *iso*-Butyl, *sec*-Butyl und *tert*-Butyl steht,
R⁴ für einen Wasserstoff-Rest, für einen Alkyl-Rest ausgewählt aus der Gruppe bestehend aus Methyl, Ethyl, *n*-Propyl, *iso*-Propyl, *n*-Butyl, *iso*-Butyl, *sec*-Butyl und *tert*-Butyl, für einen Cyclopropyl-, Cyclobutyl-, Cyclopentyl-, Cyclohexyl-, Cycloheptyl oder Cylcooctyl-Rest oder für einen Benzyl-Rest steht,
R⁵ für einen Alkyl-Rest ausgewählt aus der Gruppe bestehend aus Methyl, Ethyl, *n*-Propyl, *iso*-Propyl, *n*-Butyl, *iso*-Butyl, *sec*-Butyl und *tert*-Butyl, für einen Cyclopropyl-, Cyclobutyl-, Cyclopentyl-, Cyclohexyl-, Cycloheptyl oder Cylcooctyl-Rest oder für einen Benzyl-Rest steht,
oder
R⁴ und R⁵ zusammen mit dem sie verbindenden Stickstoffatom als Ringglied einen Imidazolidinyl-, Aziridinyl-, Azetidinyl-, Pyrrolidinyl-, Piperidinyl-, Azepanyl-, Azokanyl-, Piperazinyl-, Morpholinyl- oder Thiomorpholinyl-Rest bilden, der mit einem Substituenten ausgewählt aus der Gruppe bestehend aus Methyl, Ethyl, *n*-Propyl, *iso*-Propyl, -C(=O)-O-CH₃, -C(=O)-O-C₂H₅, - C(=O)-O-(*n*-C₃H₇), -C(=O)-O-(*iso*-C₃H₇) und ggf. über eine -(CH₂)-Gruppe gebundenem Phenyl substituiert sein kann.

8. Verbindungen gemäß einem oder mehreren der Ansprüche 1 bis 7 ausgewählt aus der Gruppe bestehend aus
1-Methyl-5-(4-methyl-piperidin-1-ylmethyl)-1H-pyrrol-2-carbonsäurebenzylmethylamid,
(5-Azepan-1-ylmethyl-1-methyl-1H-pyrrol-2-yl)-pyrrolidin-1-yl-methanon,
[1-Methyl-5-(2-methyl-piperidin-1-ylmethyl)-1H-pyrrol-2-yl]-pyrrolidin-1-yl-methanon,
1-Methyl-5-pyrrolidin-1-ylmethyl-1H-pyrrol-2-carbonsäurebenzylmethylamid,
1-Methyl-5-(2-methyl-piperidin-1-ylmethyl)-1H-pyrrol-2-carbonsäurebenzyl-methylamid,
1-(5-Dibenzylcarbamoyl-1-methyl-1H-pyrrol-2-ylmethyl)-piperidin-4-carbonsäureethylester,
5-(4-Benzyl-piperidin-1-ylmethylyl-mothyl-1H-pyrrol-2-carbonsäurecyclohexylmethylamid,
[5-(4-Benzyl-piperidin-1-ylmethyl)-1-methyl-1H-pyrrol-2-yl]-piperidin-1-yl-methanon,
1-Methyl-5-(2-methyl-piperidin-1-ylmethyl)-1H-pyrrol-2-carbonsäurebenzylisopropylamid und
[1-Methyl-5-(2-methyl-piperidin-1-ylmethyl)-1H-pyrrol-2-yl]-piperidin-1-yl-methanon,
jeweils gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren, Diastereomeren und/oder Rotameren, in einem beliebigen Mischungsverhältnis, oder jeweils in Form entsprechender Salze, oder jeweils in Form entsprechender Solvate.

9. Verfahren zur Herstellung von substituierten 5-Aminomethyl-1H-pyrrol-2-carbonsäureamiden gemäß einem oder mehreren der Ansprüche 1 bis 8, **dadurch gekennzeichnet, daß** ein substituiertes 1H-Pyrrol-2-carbonsäureamid der allgemeinen Formel II, 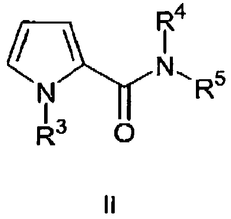 worin die Reste R³, R⁴ und R⁵ die Bedeutung gemäß einem oder mehreren der Ansprüche 1 bis 8, haben mit einem Iminiumsalz der allgemeinen Formel III, 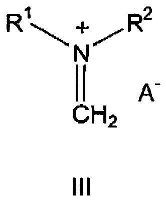 worin die Reste R¹ und R² die Bedeutung gemäß einem oder mehreren der Ansprüche 1 bis 8 haben und A⁻ für ein geeignetes Anion, vorzugsweise Cl⁻, AlCl₄⁻, Br⁻, I⁻ oder CF₃-SO₃⁻ (Triflat-Anion) steht, umgesetzt und die so erhaltene Verbindung ggf. gereinigt und ggf. isoliert wird.

10. Arzneimittel enthaltend wenigstens eine Verbindung gemäß einem oder mehreren der Ansprüche 1 bis 8 sowie ggf. einen oder mehrere physiologisch verträgliche Hilfsstoffe.

11. Arzneimittel gemäß Anspruch 10 zur Behandlung von Schmerzen.

12. Arzneimittel gemäß Anspruch 11 zur Behandlung von Schmerzen ausgewählt aus der Gruppe bestehend aus akuten Schmerzen, chronischen Schmerzen und neuropathischen Schmerzen.

13. Arzneimittel gemäß Anspruch 10 zur Prophylaxe und/oder Behandlung von Entzugserscheinungen, Gedächtnisstörungen, neurodegenerativen Erkrankungen, vorzugsweise ausgewählt aus der Gruppe bestehend aus Morbus Parkinson, Morbus Huntington, Morbus Alzheimer und Multipler Sklerose, Epilepsie, cardiovaskulären Störungen, Wasserspeicher-Krankheiten, intestinaler Motilität (Diarrhoe), Harninkontinenz, Anorexie, Tinnitus, Pruritus, Depressionen, sexueller Dysfunktionen, vorzugsweise erektiler Dysfunktion oder Atemwegserkrankungen, zur Prophylaxe und/oder Behandlung von Störungen der Nahrungsmittelaufnahme, vorzugsweise ausgewählt aus der Gruppe bestehend aus Fettsucht, Bulimie, Anorexie, Kachexie und Type II Diabetes (nicht-insutinabhangiger Diabetes), oder zur Anxiolyse, zur Diurese, zur Unterdrückung des Miktionsreflexes, zur Reduzierung des Suchtpotentials von Opioiden, vorzugsweise Morphin, zur Modulation der Bewegungsaktivität, zur Beeinflussung des cardiovaskulären Systems, vorzugsweise zur Vasodilatation der Arterien, oder zur Regulation des Elektrolyt-Haushaltes.

14. Verwendung wenigstens einer Verbindung gemäß einem oder mehreren der Ansprüche 1 bis 8 zur Herstellung eines Arzneimittels zur Behandlung von Schmerzen, vorzugsweise von Schmerzen ausgewählt aus der Gruppe bestehend aus akuten Schmerzen, chronischen Schmerzen und neuropathischen Schmerzen.

15. Verwendung wenigstens einer Verbindung gemäß einem oder mehreren der Ansprüche 1 bis 8 zur Herstellung eines Arzneimittels zur Prophylaxe und/oder Behandlung von Entzugserscheinungen, Gedächtnisstörungen, neurodegenerativen Erkrankungen, vorzugsweise ausgewählt aus der Gruppe bestehend aus Morbus Parkinson, Morbus Huntington, Morbus Alzheimer und Multipler Sklerose, Epilepsie, cardiovaskulären Störungen, Wasserspeicher-Krankheiten, intestinaler Motilität (Diarrhoe), Harninkontinenz, Anorexie, Tinnitus, Pruritus, Depressionen, sexueller Dysfunktionen, vorzugsweise erektiler Dysfunktion oder Atemwegserkrankungen, zur Prophylaxe und/oder Behandlung von Störungen der Nahrungsmittelaufnahme, vorzugsweise ausgewählt aus der Gruppe bestehend aus Fettsucht, Bulimie, Anorexie, Kachexie und Type II Diabetes (nicht-insulinabhängiger Diabetes), oder zur Anxiolyse, zur Diurese, zur Unterdrückung des Miktionsreflexes, zur Reduzierung des Suchtpotentials von Opioiden, vorzugsweise Morphin, zur Modulation der Bewegungsaktivität, zur Beeinflussung des cardiovaskulären Systems, vorzugsweise zur Vasodilatation der Arterien, oder zur Regulation des Elektrolyt-Haushaltes.

## Claims

1. Substituted 5-aminomethyl-1H-pyrrole-2-carboxylic acid amides of the general formula I, 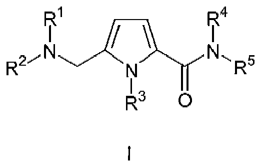 in which
residues R¹ and R², together with the nitrogen atom joining them together as a ring member, form a saturated or unsaturated 4-, 5-, 6-, 7-, 8- or 9-membered cycloaliphatic residue,
residue R³ denotes a linear or branched, unsubstituted C₁₋₅ alkyl residue, a saturated or unsaturated, unsubstituted or at least monosubstituted 4-, 5-, 6-, 7-, 8- or 9-membered cycloaliphatic residue, which may be attached via a linear or branched C₁₋₃ alkylene group or denotes an unsubstituted or at least monosubstituted 5- or 6-membered aryl or heteroaryl residue, which may be attached via a linear or branched C₁₋₃ alkylene group,
residue R⁴ denotes a hydrogen residue, a linear or branched, unsubstituted C₁₋₅ alkyl residue, an unsaturated or saturated, unsubstituted or at least monosubstituted 3-, 4-, 5-, 6-, 7-, 8- or 9-membered cycloaliphatic residue, or denotes an unsubstituted or at least monosubstituted phenyl group attached via a linear or branched C₁₋₃ alkylene group,
residue R⁵ denotes a linear or branched, unsubstituted C₁₋₅ alkyl residue, an unsaturated or saturated, unsubstituted or at least monosubstituted 3-, 4-, 5-, 6-, 7-, 8-or 9-membered cycloaliphatic residue, or denotes an unsubstituted or at least monosubstituted phenyl residue attached via a linear or branched C₁₋₃ alkylene group, or residues R⁴ and R⁵, together with the nitrogen atom joining them together as a ring member, form a saturated or unsaturated 4-, 5-, 6-, 7-, 8- or 9-membered monocyclic cycloaliphatic residue optionally comprising at least one further heteroatom selected from the group consisting of nitrogen, oxygen and sulphur as a ring member,
wherein
the corresponding substituents of the abovementioned aryl residues can be respectively selected independently of one another from the group consisting of halogen, hydroxy, -CN, -CF₃, -CHF₂, -CH₂F, -NO₂, -NH₂, -NH-C₁₋₅ alkyl, -N(C₁₋₅ alkyl)₂, -C(=O)-NH₂, - C(=O)-NH-C₁₋₅ alkyl, -C(=O)-N(C₁₋₅ alkyl)₂, C₁₋₅ alkyl, C₁₋₅ alkoxy, and optionally at least monosubstituted phenyl, wherein where the phenyl substituent is itself mono- or polysubstituted, its substituents can be selected from the group consisting of hydroxy, F, Cl, Br, I, -NH₂, -NH-C₁₋₅ alkyl, N(C₁₋₅ alkyl)₂, C(=O)-NH₂, -C(=O)-NH-C₁₋₅ alkyl, - C(=O)-N(C₁₋₅ alkyl)₂, NO₂, -CN, -CF₃, -CHF₂, -CH₂F, C₁₋₅ alkyl and C₁₋₅ alkoxy,
the corresponding substituents of the abovementioned heteroaryl residues can be respectively selected independently of one another from the group consisting of halogen, hydroxy, -CN, -CF₃, -CHF₂, -CH₂F, -NO₂, -NH₂, -NH-C₁₋₅ alkyl, -N(C₁₋₅ alkyl)₂, -C(=O)-NH₂, -C(=O)-NH-C₁₋₅ alkyl, -C(=O)-N(C₁₋₅ alkyl)₂, C₁₋₅ alkyl, C₁₋₅ alkoxy, and optionally at least monosubstituted phenyl, wherein where the phenyl substituent is itself mono- or polysubstituted, its substituents can be selected from the group consisting of hydroxy, F, Cl, Br, I, -NH₂, -NH-C₁₋₅ alkyl, N(C₁₋₅ alkyl)₂, C(=O)-NH₂, -C(=O)-NH-C₁₋₅ alkyl, -C(=O)-N(C₁₋₅ alkyl)₂, NO₂, -CN, -CF₃, -CHF₂, -CH₂F, C₁₋₅ alkyl and C₁₋₅ alkoxy, and
the abovementioned cycloaliphatic residues may be identically or differently mono- or polysubstituted with a substituent selected from the group consisting of C₁₋₅ alkyl, - C(=O)-O-C₁₋₅-alkyl, -O-C₁₋₅-alkyl, Cl, F, Br, I, -C(=O)-NH₂, -C(=O)-NH-C₁₋₅-alkyl, - C(=O)-N(C₁₋₅-alkyl)₂, -C(=O)-C₁₋₅-alkyl, -NH₂, -NH-C₁₋₅-alkyl, -N(C₁₋₅-alkyl)₂, furanyl, thiophenyl, pyridinyl, phenyl and benzyl, wherein the cyclic substituents may themselves be unsubstituted or at least monosubstituted with substituents selected independently from one another from the group consisting of hydroxy, F, Cl, Br, I, -NH₂, -NH-C₁₋₅ alkyl, N(C₁₋₅ alkyl)₂, C(=O)-NH₂, -C(=O)-NH-C₁₋₅ alkyl, -C(=O)-N(C₁₋₅ alkyl, NO₂, -CN, -CF₃, -CHF₂, -CH₂F, C₁₋₅ alkyl and C₁₋₅ alkoxy;
in each case optionally in the form of the pure stereoisomers thereof, in particular enantiomers or diastereomers, the racemates thereof or in the form of mixtures of the stereoisomers, in particular the enantiomers, diastereomers and/or rotamers, in any desired mixing ratio, or in each case in the form of corresponding salts or in each case in the form of corresponding solvates.

2. Compounds according to claim 1, **characterised in that** residues R¹ and R², together with the nitrogen atom joining them together as a ring member form an aziridinyl , azetidinyl, pyrrolidinyl, piperidinyl, azepanyl or azocanyl residue, which may be identically or differently mono- or polysubstituted with a substituent selected from the group consisting of C₁₋₅ alkyl, -C(=O)-O-C₁₋₅-alkyl, -O-C₁₋₅-alkyl, Cl, F, Br, I, - C(=O)-NH_{2.} -C(=O)-NH-C₁₋₅-alkyl, -C(=O)-N(C₁₋₅-alkyl)₂, -C(=O)-C₁₋₅-alkyl, -NH₂, - NH-C₁₋₅-alkyl, -N(C₁₋₅-alkyl)₂, furanyl, thiophenyl, pyridinyl, phenyl, and benzyl, wherein the cyclic substituents may themselves in each case be unsubstituted or at least monosubstituted.

3. Compounds according to claim 1 or 2, **characterised in that** residue R³ denotes an alkyl residue selected from the group consisting of methyl, ethyl, n-propyl, iso-propyl, n-butyl , iso-butyl, sec-butyl and tert-butyl.

4. Compounds according to one or more of claims I to 3, **characterised in that** residue R⁴ denotes a hydrogen residue, an alkyl residue selected from the group consisting of methyl, ethyl, *n*-propyl, *iso*-propyl, *n*-butyl, *iso*-butyl, *sec*-butyl and *tert*-butyl, a cyclopropyl, cyclobutyl, cyclopentyl, cyclohexyl, cycloheptyl or cyclooctyl residue or denotes an unsubstituted or at least monosubstituted benzyl residue.

5. Compounds according to one or more of claims 1 to 4, **characterised in that** residue R⁵ denotes an alkyl residue selected from the group consisting of methyl, ethyl, n-propyl, *iso*-propyl, *n*-butyl, *iso*-butyl, *sec*-butyl and *tert*-butyl, a cyclopropyl, cyclobutyl, cyclopentyl, cyclohexyl, cycloheptyl or cyclooctyl residue or denotes an unsubstituted or at least monosubstituted benzyl residue.

6. Compounds according to one or more of claims 1 to 5, **characterised in that** residues R⁴ and R⁵, together with the nitrogen atom joining them together as a ring member, form an imidazolidinyl, aziridinyl, azetidinyl, pyrrolidinyl, piperidinyl, azepanyl, azocanyl, piperazinyl, morpholinyl or thiomorpholinyl residue, which residue may be identically or differently mono- or polysubstituted with a substituent selected from the group consisting of C₁₋₅ alkyl, -C(=O)-O-C₁₋₅-alkyl, -O-C₁₋₅-alkyl, Cl, F, Br, I, - C(=O)-NH₂, -C(=O)-NH-C₁₋₅-alkyl, -C(=O)-N(C₁₋₅-alkyl)₂, -C(=O)-C₁₋₅-alkyl, -NH₂, - NH-C₁₋₅-alkyl, -N(C₁₋₅-alkyl)₂, furanyl, thiophenyl, pyridinyl, phenyl and benzyl, wherein the cyclic substituents may themselves in each case be unsubstituted or at least monosubstituted.

7. Compounds according to one or more of claims I to 6, **characterised in that**
R¹ and R², together with the nitrogen atom joining them together as a ring member, form an aziridinyl, azetidinyl, pyrrolidinyl, piperidinyl, azepanyl or azocanyl residue, which may be substituted with a substituent selected from the group consisting of methyl, ethyl, *n*-propyl, *iso*-propyl, -C(=O)-O-CH₃, -C(=O)-O-C₂H₅, -C(=O)-O-(*n-*C₃H₇), -C(=O)-O-(*iso*-C₃H₇) and phenyl optionally attached via a -(CH₂) group,
R³ denotes an alkyl residue selected from the group consisting of methyl, ethyl, *n-*propyl, *iso*-propyl, *n*-butyl, *iso*-butyl, *sec*-butyl and *tert*-butyl,
R⁴ denotes a hydrogen residue, an alkyl residue selected from the group consisting of methyl, ethyl, *n*-propyl, *iso*-propyl, *n*-butyl, *iso*-butyl, *sec*-butyl and *tert*-butyl, a cyclopropyl, cyclobutyl, cyclopentyl, cyclohexyl, cycloheptyl or cyclooctyl residue or denotes a benzyl residue,
R⁵ denotes an alkyl residue selected from the group consisting of methyl, ethyl, n-propyl, *iso*-propyl, *n*-butyl, *iso*-butyl, *sec*-butyl and *tert*-butyl, a cyclopropyl, cyclobutyl, cyclopentyl, cyclohexyl, cycloheptyl or cyclooctyl residue or denotes a benzyl residue,
or
R⁴ and R⁵, together with the nitrogen atom joining them together as a ring member, form an imidazolidinyl, aziridinyl, aretidinyl, pyrrolidinyl, piperidinyl, azepanyl, azocanyl, piperazinyl, morpholinyl or thiomorpholinyl residue, which may be substituted with a substituent selected from the group consisting of methyl, ethyl, n-propyl, *iso*-propyl, -C(=O)-O-CH₃, -C(=O)-O-C₂H₅, -C(=O)-O-(*n*-C₃H₇), -C(=O)-O-(*iso*-C₃H₇) and phenyl optionally attached via a -(CH₂) group.

8. Compounds according to one or more of claims 1 to 7 selected from the group consisting of
1-methyl-5-(4-methyl-piperidin-1-ylmethyl)-1H-pyrrole-2-carboxylic acid benzylmethyl amide,
(5-azepan-1-ylmethyl-1-methyl-1H-pyrrol-2-yl)-pyrrolidin-1-yl methanone,
[1-methyl-5-(2-methyl-piperidin-1-ylmethyl)-1H-pyrrol-2-yl]-pyrrolidin-1-yl methanone,
1-methyl-5-pyrrolidin-1-ylmethyl-1H-pyrrole-2-carboxylic acid benzylmethyl amidc,
1-methyl-5-(2-methyl-piperidin-I-ylmethyl)-1H-pyrrole-2-carboxylic acid benzylmethyl amide,
1-(5-dibenzylcarbamoyl-1-methyl-1H-pyrrol-2-ylmethyl)-piperidine-4-carboxylic acid ethyl ester,
5-(4-benzyl-piperidin-1-ylmethyl)-1-methyl-1H-pyrrole-2-carboxylic acid cyclohexylmethyl amide,
[5-(4-benzyl-piperidin-1-ylmethyl)-1-methyl-1H-pyrrol-2-yl]-piperidin-1-yl methanone,
1-methyl-5-(2-methyl-piperidin-1-ylmethyl)-1H-pyrrole-2-carboxylic acid benzylisopropyl amide and
[1-methyl-5-(2-methyl-piperidin-1-ylmethyl)-1H-pyrrol-2-yl]-piperidin-1-yl methanone,
in each case optionally in the form of the pure stereoisomers thereof, in particular enantiomers or diastereomers, the racemates thereof or in the form of mixtures of the stereoisomers, in particular the enantiomers, diastereomers and/or rotamers, in any desired mixing ratio, or in each case in the form of corresponding salts or in each case in the form of corresponding solvates.

9. A process for the production of substituted 5-aminomethyl-1H-pyrrole-2-carboxylic acid amides according to one or more of claims 1 to 8, **characterised in that** a substituted 1H-pyrrole-2-carboxylic acid amide of the general formula II, 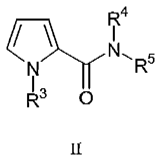 in which residues R³, R⁴ and R⁵ have the meaning according to one or more of claims 1 to 8, is reacted with an iminium salt of the general formula III, 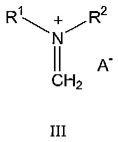 in which residues R¹ and R² have the meaning according to one or more of claims 1 to 8, and A⁻ denotes a suitable anion, preferably Cl⁻, AlCl₄⁻, Br⁻, I⁻ or CF₃-SO₃⁻ (triflate anion) and the resultant compound is optionally purified and optionally isolated.

10. A pharmaceutical preparation containing at least one compound according to one or more of claims 1 to 8 and optionally one or more physiologically acceptable auxiliary substances.

11. A pharmaceutical preparation according to claim 10 for the treatment ofpain.

12. A pharmaceutical preparation according to claim 11 for the treatment ofpain selected from the group consisting of acute pain, chronic pain, and neuropathic pain.

13. A pharmaceutical preparation according to claim 10 for the prevention and/or treatment of withdrawal symptoms, memory disorders, neurodegenerative diseases, preferably selected from the group consisting of Parkinson's disease, Huntington's disease, Alzheimer's disease and multiple sclerosis, epilepsy, cardiovascular disorders, water retention conditions, intestinal motility (diarrhoea), urinary incontinence, anorexia, tinnitus, pruritus, depression, sexual dysfunction, preferably erectile dysfunction, or respiratory tract diseases, for the prevention and/or treatment of eating disorders preferably selected from the group consisting of obesity, bulimia, anorexia, cachexia and type II diabetes (non-insulin-dependent diabetes), or for anxiolysis, for diuresis, for suppressing the urinary reflex, for reducing the addictive potential of opioids, preferably morphine, for modulating mobility, for influencing the cardiovascular system, preferably for vasodilating the arteries, or for regulating the electrolyte balance.

14. Use of at least one compound according to one or more the claims I to 8 for the production of a pharmaceutical preparation for the treatment of pain, preferably of pain selected from the group consisting of acute pain, chronic pain, and neuropathic pain.

15. Use of at least one compound according to one or more the claims 1 to 8 for the production of a pharmaceutical preparation for the prevention and/or treatment of withdrawal symptoms, memory disorders, neurodegenerative diseases, preferably selected from the group consisting of Parkinson's disease, Huntington's disease, Alzheimer's disease and multiple sclerosis, epilepsy, cardiovascular disorders, water retention conditions, intestinal motility (diarrhoea), urinary incontinence, anorexia, tinnitus, pruritus, depression, sexual dysfunction, preferably erectile dysfunction, or respiratory tract diseases, for the prevention and/or treatment of eating disorders, preferably selected from the group consisting of obesity, bulimia, anorexia, cachexia and type II diabetes (non-insulin-dependent diabetes), or for anxiolysis, for diuresis, for suppression of the urinary reflex, for reducing the addictive potential of opioids, preferably morphine, for modulating mobility, for influencing the cardiovascular system, preferably for vasodilating the arteries, or for regulating the electrolyte balance.

## Revendications

1. Amides de l'acide 5-aminométhyl-1H-pyrrol-2-carboxylique substitués de formule générale I 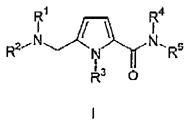 dans laquelle
les radicaux R¹ et R² forment ensemble avec l'atome d'azote qui les relie en tant qu'élément de cycle un radical cycloaliphatique saturé ou insaturé à 4, 5, 6, 7, 8 ou 9 éléments ;
le radical R³ représente un radical alkyle en C₁₋₅ non substitué linéaire ou ramifié, un radical cycloaliphatique saturé ou insaturé, non substitué ou substitué au moins une fois, à 4, 5, 6, 7, 8 ou 9 éléments, qui peut être relié par un groupe alkylène en C₁₋₃ linéaire ou ramifié, ou un radical aryle ou hétéroaryle non substitué ou substitué au moins une fois, à 5 ou 6 éléments, qui peut être relié par un groupe alkylène en C₁₋₃ linéaire ou ramifié,
le radical R⁴ représente un radical hydrogène, un radical alkyle en C₁₋₅ non substitué, linéaire ou ramifié, un radical cycloaliphatique insaturé ou saturé, non substitué ou substitué au moins une fois, à 3, 4, 5, 6, 7, 8 ou 9 éléments, ou un radical phényle non substitué ou substitué au moins une fois, relié par un groupe alkylène en C₁₋₃ linéaire ou ramifié,
le radical R⁵ représente un radical alkyle en C₁₋₅ non substitué, linéaire ou ramifié, un radical cycloaliphatique insaturé ou saturé, non substitué ou substitué au moins une fois, à 3, 4, 5, 6, 7, 8 ou 9 éléments, ou un radical phényle non substitué ou substitué au moins une fois, relié par un groupe alkylène en C₁₋₃ linéaire ou ramifié,
ou les radicaux R⁴ et R⁵ forment ensemble avec l'atome d'azote qui les relie en tant qu'élément de cycle un radical cycloaliphatique monocyclique saturé ou insaturé à 4, 5, 6, 7, 8 ou 9 éléments, comprenant éventuellement au moins un hétéroatome supplémentaire choisi dans le groupe constitué par l'azote, l'oxygène et le soufre en tant qu'élément de cycle,
les substituants correspondants des radicaux aryle susmentionnés pouvant à chaque fois être choisis indépendamment les uns des autres dans le groupe constitué par halogène, hydroxy, -CN, -CF₃, -CHF₂, - CH₂F, -NO2, -NH₂, -NH-alkyle en C₁₋₅, -N(alkyle en C₁₋₅)₂, -C(=O)-NH₂, -C(=O)-NH-alkyle en C₁₋₅, -C(=O)-N(alkyle en C₁₋₅)₂, alkyle en C₁₋₅, alcoxy en C₁₋₅ et phényle éventuellement substitué au moins une fois, si le substituant phényle est lui-même substitué une ou plusieurs fois, ses substituants pouvant être choisis dans le groupe constitué par hydroxy, F, Cl, Br, I, - NH₂, -NH-alkyle en C₁₋₅, -N(alkyle en C₁₋₅)₂, -C(=)-NH₂, -C(=O)-NH-alkyle en C₁₋₅, -C(=O)-N(alkyle en C₁₋₅)₂, -NO₂, -CN, -CF₃, -CHF₂, -CH₂F, alkyle en C₁₋₅ et alcoxy en C₁₋₅, les substituants correspondants des radicaux hétéroaryle susmentionnés pouvant à chaque fois être choisis indépendamment les uns des autres dans le groupe constitué par halogène, hydroxy, -CN, -CF₃,-CH₂, -CH₂F, -NO₂, -NH₂, -NH-alkyle en C₁₋₅, -N(alkyle en C₁₋₅)₂, -C(=O)-NH₂, -C(=O)-NH-alkyle en C₁₋₅, -C(=O)-N(alkyle en C₁₋₅)₂, alkyle en C₁₋₅, alcoxy en C₁₋₅ et phényle éventuellement substitué au moins une fois, si le substituant phényle est lui-même substitué une ou plusieurs fois, ses substituants pouvant être choisis dans le groupe constitué par hydroxy, F, Cl, Br, I, - NH₂, -NH-alkyle en C₁₋₅, -N(alkyle en C₁₋₅)₂, -C(=O)-NH₂, -C(=O)-NH-alkyle en C₁₋₅, -C(=O)-N(alkyle en C₁₋₅)₂, -NO₂, -CN, -CF₃, -CHF₂, -CH₂F, alkyle en C₁₋₅ et alcoxy en C₁₋₅, et
les radicaux cycloaliphatiques susmentionnés pouvant être substitués une ou plusieurs fois, de manière identique ou différente, avec un substituant choisi dans le groupe constitué par alkyle en C₁₋₅, -C(=O)-O-alkyle en C₁₋₅, -O-alkyle en C₁₋₅, Cl, F, Br, I, -C(=O)-NH₂, -C(=O)-NH-alkyle en C₁₋₅, -C(=O)-N(alkyle en C₁₋₅)₂, -C(=O)-alkyle en C₁₋₅, -NH₂, -NH-alkyle en C₁₋₅, -N(alkyle en C₁₋₅)₂, furanyle, thiophényle, pyridinyle, phényle et benzyle, les substituant cycliques pouvant eux-mêmes à chaque fois être non substitués ou substitués au moins une fois avec des substituants choisis indépendamment les uns des autres dans le groupe constitué par hydroxy, F, Cl, Br, I, -NH₂, -NH-alkyle en C₁₋₅,-N (alkyle en C₁₋₅)₂, -C(=O)-NH₂, -C(=O)-NH-alkyle en C₁₋₅, -C(=O)-N(alkyle en C₁₋₅)₂, -NO₂, -CN, -CF₃, -CHF₂, -CH₂F, alkyle en C₁₋₅ et alcoxy en C₁₋₅;
à chaque fois éventuellement sous la forme de leurs stéréoisomères purs, notamment énantiomères ou diastéréomères, de leurs racémates ou sous la forme de mélanges des stéréoisomères, notamment des énantiomères, diastéréomères et/ou rotamères, en un rapport de mélange quelconque, ou à chaque fois sous la forme des sels correspondants, ou à chaque fois sous la forme des solvates correspondants.

2. Composés selon la revendication 1, **caractérisés en ce que** les radicaux R¹ et R² forment ensemble avec l'atome d'azote qui les relie en tant qu'élément de cycle un radical aziridinyle, azétidinyle, pyrrolidinyle, pipéridinyle, azépanyle ou azocanyle, qui peut être substitué une ou plusieurs fois, de manière identique ou différente, avec un substituant choisi dans le groupe constitué par alkyle en C₁₋₅, C(=O)-O-al-kyle en C₁₋₅, -O-alkyle en C₁₋₅, Cl, F, Br, I, -C(=O)-NH₂, -C(=O)-NH-alkyle en C₁₋₅, -C(=O)-N(alkyle en C₁₋₅)₂, -C(=O)-alkyle en C₁₋₅, -NH₂, -NH-alkyle en C₁₋₅,-N(alkyle en C₁₋₅)₂, furanyle, thiophényle, pyridinyle, phényle et benzyle, les substituants cycliques pouvant eux-mêmes à chaque fois être non substitués ou substitués au moins une fois.

3. Composés selon la revendication 1 ou 2, **caractérisés en ce que** le radical R³ représente un radical alkyle choisi dans le groupe constitué par méthyle, éthyle, *n*-propyle, *iso*-propyle, *n*-butyle, *iso-*butyle, *sec*-butylc et *tert*-butyle.

4. Composés selon une ou plusieurs des revendications 1 à 3, **caractérisés en ce que** le radical R⁴ représente un radical hydrogène, un radical alkyle choisi dans le groupe constitué par méthyle, éthyle, *n*-propyle, *iso*-propyle, *n*-butyle, *iso*-butyle, *sec*-butyle et *tert*-butyle, un radical cyclopropyle, cyclobutyle, cyclopentyle, cyclohexyle, cycloheptyle ou cyclooctyle, ou un radical benzyle non substitué ou substitué au moins une fois.

5. Composés selon une ou plusieurs des revendications 1 à 4, **caractérisés en ce que** le radical R⁵ représente un radical alkyle choisi dans le groupe constitué par méthyle, éthyle, *n*-propyle, *iso-*propyle, *n*-butyle, *iso*-butyle, *sec*-butyle et *tert-*butyle, un radical cyclopropyle, cyclobutyle, cyclopentyle, cyclohexyle, cycloheptyle ou cyclooctyle, ou un radical benzyle non substitué ou substitué au moins une fois.

6. Composés selon une ou plusieurs des revendications 1 à 5, **caractérisés en ce que** les radicaux R⁴ et R⁵ forment ensemble avec l'atome d'azote qui les relie en tant qu'élément de cycle un radical imidazolidinyle, aziridinyle, azétidinyle, pyrrolidinyle, pipéridinyle, azépanyle, azocanyle, pipérazinyle, morpholinyle ou thiomorpholiny7.e, qui peut être substitué une ou plusieurs fois, de manière identique ou différente, avec un substituant choisi dans le groupe constitué par alkyle en C₁₋₅, -C(=O)-O-alkyle en C₁₋₅, -O-alkyle en C₁₋₅, Cl, F, Br, I, -C(=O)-NH₂, -C(=O)-NH-alkyle en C₁₋₅, -C(=O)-N(alkyle en C₁₋₅)₂, -C(=0)-alkyle en C₁₋₅, -NH₂, -NH-alkyle en C₁₋₅, -N(alkyle en C₁₋₅)₂, furanyle, thiophényle, pyridinyle, phényle et benzyle, les substituants cycliques pouvant eux-mêmes à chaque fois être non substitués ou substitués au moins une fois.

7. Composés selon une ou plusieurs des revendications 1 à 6, **caractérisés en ce que** R¹ et R² forment ensemble avec l'atome d'azote qui les relie en tant qu'élément de cycle un radical aziridinyle, azétidinyle, pyrrolidinyle, pipéridinyle, azépanyle ou azocanyle, qui peut être substitué avec un substituant choisi dans le groupe constitué par méthyle, éthyle, *n*-propyle, *iso*-propyle, -C(=O)-O-CH₃, - C(=O)-O-C₂H₅, -C(=O)-O-(*n*-C₃H₇), -C(=O)-O-(*iso*-C₃H₇), et phényle éventuellement relié par un groupe (CH₂),
R³ représente un radical alkyle choisi dans le groupe constitué par méthyle, éthyle, *n*-propyle, *iso*-propyle, *n*-butyle, *iso*-butyle, *sec*-butyle et *tert*-butyle,
R⁴ représente un radical hydrogène, un radical alkyle choisi dans le groupe constitué par méthyle, éthyle, *n-*propyle, *iso*-propyle, *n*-butyle, *iso*-butyle, *sec*-butyle, et *tert*-butyle, un radical cyclopropyle, cyclobutyle, cyclopentyle, cyclohexyle, cycloheptyle ou cyclooctyle, ou un radical benzyle,
R' représente un radical alkyle choisi dans le groupe constitué par méthyle, éthyle, *n*-propyle, *iso*-propyle, *n*-butyle, *iso*-butyle, *sec*-butyle et *tert*-butyle, un radical cyclopropyle, cyclobutyle, cyclopentyle, cyclohexyle, cycloheptyle ou cyclooctyle, ou un radical benzyle,
ou
R⁴ et R⁵ forment ensemble avec l'atome d'azote qui les relie en tant qu'élément de cycle un radical imidazolidinyle, aziridinyle, azétidinyle, pyrrolidinyle, pipéridinyle, azépanyle, azocanyle, pipérazinyle, morpholinyle ou thiomorpholinyle, qui peut être substitué avec un substituant choisi dans le groupe constitué par méthyle, éthyle, *n*-propyle, *iso-*propyle, -C(=O)-O-CH₃, -C(=O)-O-C₂H₅, -C(=O)-O-(*n*-C₃H₇), -C(=O)-O-(*iso*-C₃H₇), et phényle éventuellement relié par un groupe (CH₂).

8. Composés selon une ou plusieurs des revendications 1 à 7, choisis dans le groupe constitué par
le benzylméthylamide de l'acide 1-méthyl-5-(4-méthyl-pipéridin-1-yl-méthyl)-1H-pyrrol-2-carboxylique,
la (5-azépan-1-ylméthyl-1-méthyl-1H-pyrrol-2-yl)-pyrrolidin-7-yl-méthanone,
la [1-méthyl-5-(2-méthyl--pipéridin-1-ylméthyl)-1H-pyrrol-2-yl]-pyrrolidin-1-yl-méthanone,
le benzylméthylamide de l'acide 1-méthyl-5-pyrrolidin-1-ylméthyl-1H-pyrrol-2-carboxylique,
le benzylméthylamide de l'acide 1-méthyl-5-(2-méthyl-pipéridin-1-ylméthyl)-1H-pyrrol-2-carboxylique,
l'ester éthylique de l'acide 1-(5-dibenzylcarbamoyl-1-méthyl-1H-pyrrol-2-ylméthyl)-pipéridine-4-carboxylique, le cyclohexylméthylamide de l'acide 5-(4-benzyl-pipéridin-1-ylméthyl)-1-méthyl-1H-pyrrol-2-carboxylique,
la [5-(4-benzyl-pipéridin-1-ylméthyl)-1-méthyl-1H-pyrrol-2-yl]-pipéridin-1-yl-méthanone,
le benzylisopropylamide de l'acide 1-méthyl-5-(2-méthyl-pipéridin-1-ylméthyl)-1H-pyrrol-2-carboxylique et
la [1-méthyl-5-(2-méthyl-pipéridin-1-ylméthyl)-1H-pyrrol-2-yl]-pipéridin-1-yl-méthanone,
à chaque fois éventuellement sous la forme de leurs stéréoisomères purs, notamment énantiomères ou diastéréomères, de leurs racémates ou sous la forme de mélanges des stéréoisomères, notamment des énantiomères, diastéréomères et/ou rotamères, en un rapport de mélange quelconque, ou à chaque fois sous la forme des sels correspondants, ou à chaque fois sous la forme des solvates correspondants.

9. Procédé de fabrication d'amides de l'acide 5-aminométhyl-1H-pyrrol-2-carboxylique substitués selon une ou plusieurs des revendications 1 à 8, **caractérisé en ce qu'**un amide de l'acide 1H-pyrrol-2-carboxylique substitué de formule générale II 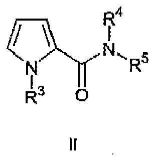 dans laquelle les radicaux R³, R⁴ et R⁵ ont la signification selon une ou plusieurs des revendications 1 à 8, est mis en réaction avec un sel d'iminium de formule générale III 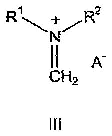 dans laquelle les radicaux R¹ et R² ont la signification selon une ou plusieurs des revendications 1 à 8 et A⁻ représente un anion approprié, de préférence Cl⁻, AlCl₄⁻, Br⁻, I⁻ ou CF₃-SO₃⁻ (anion triflate), et le composé ainsi obtenu est éventuellement purifié et éventuellement isolé.

10. Médicament contenant au moins un composé selon une ou plusieurs des revendications 1 à 8, ainsi qu'éventuellement un ou plusieurs adjuvants physiologiquement compatibles.

11. Médicament selon la revendication 10 pour le traitement de la douleur.

12. Médicament selon la revendication 11 pour le traitement de la douleur choisie dans le groupe constitué par la douleur aiguë, la douleur chronique et la douleur neuropathique.

13. Médicament selon la revendication 10 pour la prophylaxie et/ou le traitement des phénomènes de sevrage, des troubles de la mémoire, des maladies neurodégénératives, de préférence choisies dans le groupe constitué par la maladie de Parkinson, la maladie d'Huntington, la maladie d'Alzheimer et la sclérose en plaques, de l'épilepsie, des troubles cardiovasculaires, des maladies de la rétention d'eau, de la motilité intestinale (diarrhée), de l'incontinence urinaire, de l'anorexie, des acouphènes, du prurit, des dépressions, des dysfonctionnements sexuels, de préférence le dysfonctionnement érectile, ou des maladies des voies respiratoires, pour la prophylaxie et/ou le traitement des troubles de l'alimentation, de préférence choisis dans le groupe constitué par l'obésité, la boulimie, l'anorexie, la cachexie et le diabète de type II (diabète non insulinodépendant), ou pour l'anxiolyse, pour la diurèse, pour la suppression du réflexe de miction, pour la réduction du potentiel d'accoutumance des opioïdes, de préférence la morphine, pour la modulation de l'activité motrice, pour la régulation du système cardiovasculaire, de préférence pour la vasodilatation des artères, ou pour la régulation de l'équilibre électrolytique.

14. Utilisation d'au moins un composé selon une ou plusieurs des revendications 1 à 8 pour la fabrication d'un médicament pour le traitement de la douleur, de préférence de la douleur choisie dans le groupe constitué par la douleur aiguë, la douleur chronique et la douleur neuropathique.

15. Utilisation d'au moins un composé selon une ou plusieurs des revendications 1 à 8 pour la fabrication d'un médicament pour la prophylaxie et/ou le traitement des phénomènes de sevrage, des troubles de la mémoire, des maladies neurodégénératives, de préférence choisies dans le groupe constitué par la maladie de Parkinson, la maladie d'Huntington, la maladie d'Alzheimer et la sclérose en plaques, de l'épilepsie, des troubles cardiovasculaires, des maladies de la rétention d'eau, de la motilité intestinale (diarrhée), de l'incontinence urinaire, de l'anorexie, des acouphènes, du prurit, des dépressions, des dysfonctionnements sexuels, de préférence le dysfonctionnement érectile, ou des maladies des voies respiratoires, pour la prophylaxie et/ou le traitement des troubles de l'alimentation, de préférence choisis dans le groupe constitué par l'obésité, la boulimie, l'anorexie, la cachexie et le diabète de type II (diabète non insulinodépendant), ou pour l'anxiolyse, pour la diurèse, pour la suppression du réflexe de miction, pour la réduction du potentiel d'accoutumance des opioïdes, de préférence la morphine, pour la modulation de l'activité motrice, pour la régulation du système cardiovasculaire, de préférence pour la vasodilatation des artères, ou pour la régulation de l'équilibre électrolytique.
